# EUROPEAN PATENT APPLICATION

(11) **EP 2 311 948 A1**
(43) Date of publication of application: **20.04.2011**
(21) Application number: 10180084.5
(22) Date of filing: 27.02.2004
(51) Int. Cl.: C12N 15/11, C12N 15/12, A61K 48/00, C12N 15/113

(54) **RNA-interference for ZNFN3A1-gene as a method for inhibiting cancer cell growth**

(30) Priority: 28.02.2003 US 450644 P
(62) Divisional of application: 04715568.4
(71) Applicant: Onco Therapy Science, Inc., Kawasaki-shi Kanagawa 213-0012 (JP)
(72) Inventor: Nakamura, Yusuke, Kanagawa 225-0011 (JP); Furukawa, Yoichi, Kanagawa 216-0033 (JP)
(74) Representative: Vossius & Partner

(57) **Abstract**

The invention features a method for inhibiting growth of a cancer cell by contacting the cell with a composition of a ZNFN3A1 siRNA. Methods of treating cancer are also within the invention. The invention also features products, including nucleic acid sequences and vectors as well as to compositions comprising them, useful in the provided methods. The invention also provides a method for inhibiting of tumer cell, for example liver or colon cancer cell, particularly HCC or colorectal adenocarcinoma.

## Description

### Technical Field

The present invention relates to the field of biological science, more specifically to the field of cancer research. In particular, the present invention relates a composition comprising a ZNFN3A1 small interfering RNA (siRNA).

### Background Art

Hepatocellular carcinoma (HCC) is among the five most frequent cancers and is the fourth leading cause of cancer death in the world. Although recent medical advances have made great progress in diagnosing the disease, a large number of patients with HCCs are still diagnosed at advanced stages. Most of the patients are not cured by surgical resection because of severe liver dysfunction, widespread and/or multiple tumors, or high incidence of recurrence. Therefore development of highly effective chemotherapeutic drugs and preventive strategies are matters of pressing concern.

### Disclosure of the Invention

The present invention based on the surprising discovery that small interfering RNAs (siRNAs) selective for ZNFN3A1 are effective for inhibiting the cellular growth of various cancer cells, including those involved in HCC.

The invention provides methods for inhibiting cell growth. Among the methods provided are those comprising contacting a cell with a composition comprising a ZNFN3A1 small interfering RNA (siRNA). The invention also provides methods for inhibiting tumor cell growth in a subject. Such methods include administering to a subject a composition comprising a ZNFN3A1 small interfering RNA (siRNA). Another aspect of the invention provides methods for inhibiting the expression of the ZNFN3A1 gene in a cell of a biological sample. Expression of the gene may be inhibited by introduction of a double stranded ribonucleic acid (RNA) molecule into the cell in an amount sufficient to inhibit expression of the ZNFN3A1 gene. Another aspect of the invention relates to products including nucleic acid sequences and vectors as well as to compositions comprising them, useful, for example, in the provided methods. Among the products provided are siRNA molecules having the property to inhibit expression of the ZNFN3A1 gene when introduced into a cell expressing said gene. Among such molecules are those that comprise a sense strand and an antisense strand, wherein the sense strand comprises a ribonucleotide sequence corresponding to a ZNFN3A1 target sequence, and wherein the antisense strand comprises a ribonucleotide sequence which is complementary to said sense strand. The sense and the antisense strands of the molecule hybridize to each other to form a double-stranded molecule.

As used herein, the term "organism" refers to any living entity comprised of at least one cell. A living organism can be as simple as, for example, a single eukaryotic cell or as complex as a mammal, including a human being.

As used herein, the term "biological sample" refers to a whole organism or a subset of its tissues, cells or component parts (e.g. body fluids, including but not limited to blood, mucus, lymphatic fluid, synovial fluid, cerebrospinal fluid, saliva, amniotic fluid, amniotic cord blood, urine, vaginal fluid and semen). "Biological sample" further refers to a homogenate, lysate, extract, cell culture or tissue culture prepared from a whole organism or a subset of its cells, tissues or component parts, or a fraction or portion thereof. Lastly, "biological sample" refers to a medium, such as a nutrient broth or gel in which an organism has been propagated, which contains cellular components, such as proteins or polynucleotides.

The invention features methods of inhibiting cell growth. Cell growth is inhibited by contacting a cell with a composition of a ZNFN3A1 small interfering RNA (siRNA). ZNFN3A1 is a zinc finger protein that is overexpressed in tumors such as hepatocellular carcinoma or colorectal adenocarcinoma. Growth of the cell expressing ZNFN3A1 can be inhibited by the present invention. The cell is further contacted with a transfection-enhancing agent. The cell is provided *in vitro, in vivo* or *ex vivo*. The subject is a mammal, *e.g*., a human, non-human primate, mouse, rat, dog, cat, horse, or cow. The cell is a hepatic cell or a colon cell. Alternatively, the cell is a tumor cell (*i.e*., cancer cell) such as a colorectal cancer cell or a liver cancer cell. For example, the cell is a colorectal adenocarcinoma cell or a hepatocellular carcinoma cell. By inhibiting cell growth is meant that the treated cell proliferates at a lower rate or has decreased viability than an untreated cell. Cell growth is measured by proliferation assays known in the art.

By the term "siRNA"' is meant a double stranded RNA molecule which prevents translation of a target mRNA. Standard techniques of introducing siRNA into the cell are used, including those in which DNA is a template from which RNA is transcribed. The siRNA includes a sense ZNFN3A1 nucleic acid sequence, an anti-sense ZNFN3A1 nucleic acid sequence or both. The siRNA is constructed such that a single transcript has both the sense and complementary antisense sequences from the target gene, *e.g*., a hairpin.

The method is used to alter gene expression in a cell in which expression of ZNFN3A1 is upregulated, *e.g*., as a result of malignant transformation of the cells. Binding of the siRNA to an ZNFN3A1 transcript in the target cell results in a reduction in ZNFN3A1 production by the cell. The length of the oligonucleotide is at least 10 nucleotides and may be as long as the naturally-occurring ZNFN3A1 transcript. Preferably, the oligonucleotide is 19-25 nucleotides in length. Most preferably, the oligonucleotide is less than 75, 50, or 25 nucleotides in length. Examples of ZNFN3A1 siRNA oligonucleotides which inhibit ZNFN3A1 expression in mammalian cells include oligonucleotides containing target sequences, for example, nucleotides 451-471, 532-552, 623-643, 625-645, 636-656,726-746, 923-943, 1065-1085, and 1258-1278 of SEQ ID NO:1.

Methods for designing double stranded RNA having the ability to inhibit gene expression in a target cell are known. (See for example, US Patent No. 6,506,559, herein incorporated by reference in its entirety). For example, a computer program for designing siRNAs is available from the Ambion website
(http://www.ambion.com/techlib/misc/siRNA_finder.html).
The computer program selects nucleotide sequences for siRNA synthesis based on the following protocol.

### Selection of siRNA Target Sites

1. Beginning with the AUG start codon of the transcript, scan downstream for AA dinucleotide sequences. Record the occurrence of each AA and the 3' adjacent 19 nucleotides as potential siRNA target sites. Tuschl et al. recommend against designing siRNA to the 5' and 3' untranslated regions (UTRs) and regions near the start codon (within 75bases) as these may be richer in regulatory protein binding sites. UTR-binding proteins and/or translation initiation complexes may interfere with binding of the siRNA endonuclease complex.
2. Compare the potential target sites to the appropriate genome database (human, mouse, rat, etc.) and eliminate from consideration any target sequences with significant homology to other coding sequences. We suggest using BLAST, which can be found on the NCBI server at: www.ncbi.nlm.nih.gov/BLAST/
3. Select qualifying target sequences for synthesis. Selecting several target sequences along the length of the gene to evaluate is typical.

Also included in the invention are isolated polynucleotides that include the nucleic acid sequence of target sequences, for example, nucleotides 451-471 (SEQ ID NO:58), 532-552 (SEQ ID NO:60), 623-643 (SEQ ID NO:61), 625-645 (SEQ ID NO:62), 636-656 (SEQ ID NO:63), 726-746 (SEQ ID NO:64), 923-943 (SEQ ID NO:66), 1065-1085 (SEQ ID NO:68), and 1258-1278 (SEQ ID NO:69) of SEQ ID NO:1 or a polynucleotide that is complementary to the nucleic acid sequence of nucleotides 451-471, 532-552, 23-643, 625-645, 636-656, 726-746, 923-943, 1065-1085, and 1258-1278 of SEQ ID NO:1. As used herein, an "isolated nucleic acid" is a nucleic acid removed from its original environment (e.g., the natural environment if naturally occurring) and thus, synthetically altered from its natural state. In the present invention, isolated nucleic acid includes DNA, RNA, and derivatives thereof. When the isolated nucleic acid is RNA or derivatives thereof, base "t" shoulde be replaced with "u" in the nucleotide sequences. As used herein, the term "complementary" refers to Watson-Crick or Hoogsteen base pairing between nucleotides units of a polynucleotide, and the term "binding" means the physical or chemical interaction between two polypeptides or compounds or associated polypeptides or compounds or combinations thereof. Complementary nucleic acid sequences hybridize under appropriate conditions to form stable duplexes containing few or no mismatches. Furthermore, the sense strand and antisense strand of the isolated nucleotide of the present invention, can form double stranded nucleotide or hairpin loop structure by the hybridization. In a preferred embodiment, such duplexes contain no more than 1 mismatch for every 10 matches, In an especially preferred embodiment, where the strands of the duplex are fully complementary, such duplexes contain no mismatches. The polynucleotide is less than 1622 nucleotides in length. For example, the polynucleotide is less than 500, 200, or 75 nucleotides in length. Also included in the invention is a vector containing one or more of the nucleic acids described herein, and a cell containing the vectors. The isolated nucleic acids of the present invention are useful for siRNA against ZNFN3A1 or DNA encoding the siRNA. When the nucleic acids are used for siRNA or coding DNA thereof, the sense strand is preferably longer than 19 nucleotides, and more preferably longer than 21 nucleotides.

The invention is based in part on the discovery that the gene encoding a zinc finger protein, ZNFN3A1 is overexpressed in hepatocellular carcinoma (HCC) compared to non-cancerous liver tissue. The ZNFN3A1 cDNA is 1622 nucleotides in length. The 1284 ORF encodes a 428-amino acid protein with a zinc finger motif. The nucleic acid and polypeptide sequences of ZNFN3A1 are shown in Tables 1 and 2. In Table 1, the 5' and 3' untranslated region is shown in italic, the start and stop codons are in bold. The subcellular localization of ZNFN3A1 protein is altered during cell cycle progression and by the density of cultured cells. ZNFN3A1 protein accumulates in the nucleus when cells are in middle to late S phase or cultured in sparse conditions. Whereas, ZNFN3A1 protein localizes in the cytoplasm as well as in the nucleus when cells are in other phases of the cell cycle or grown in a dense condition. ZNFN3A1 forms a ternary complex with KIAA0054 protein and RNA polymerase II *in vivo,* which activates transcription of downstream genes including epidermal growth factor receptor (*EGFR*) through a direct binding of the complex with an element of "5' -CCCTCC-3'" in the 5' flanking region.

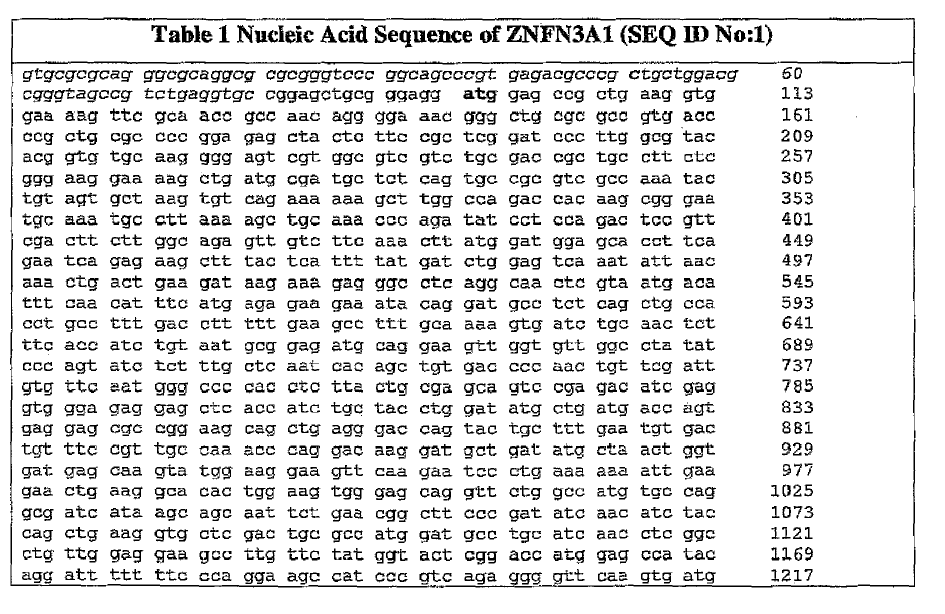

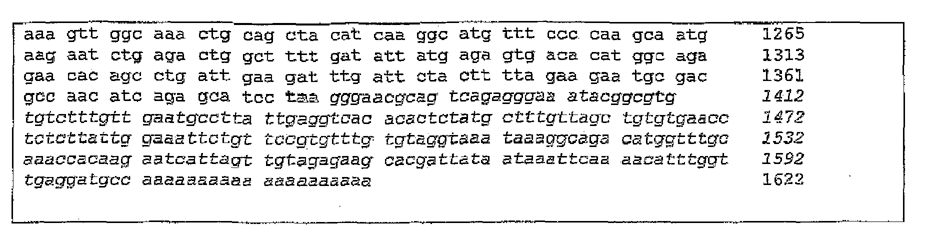

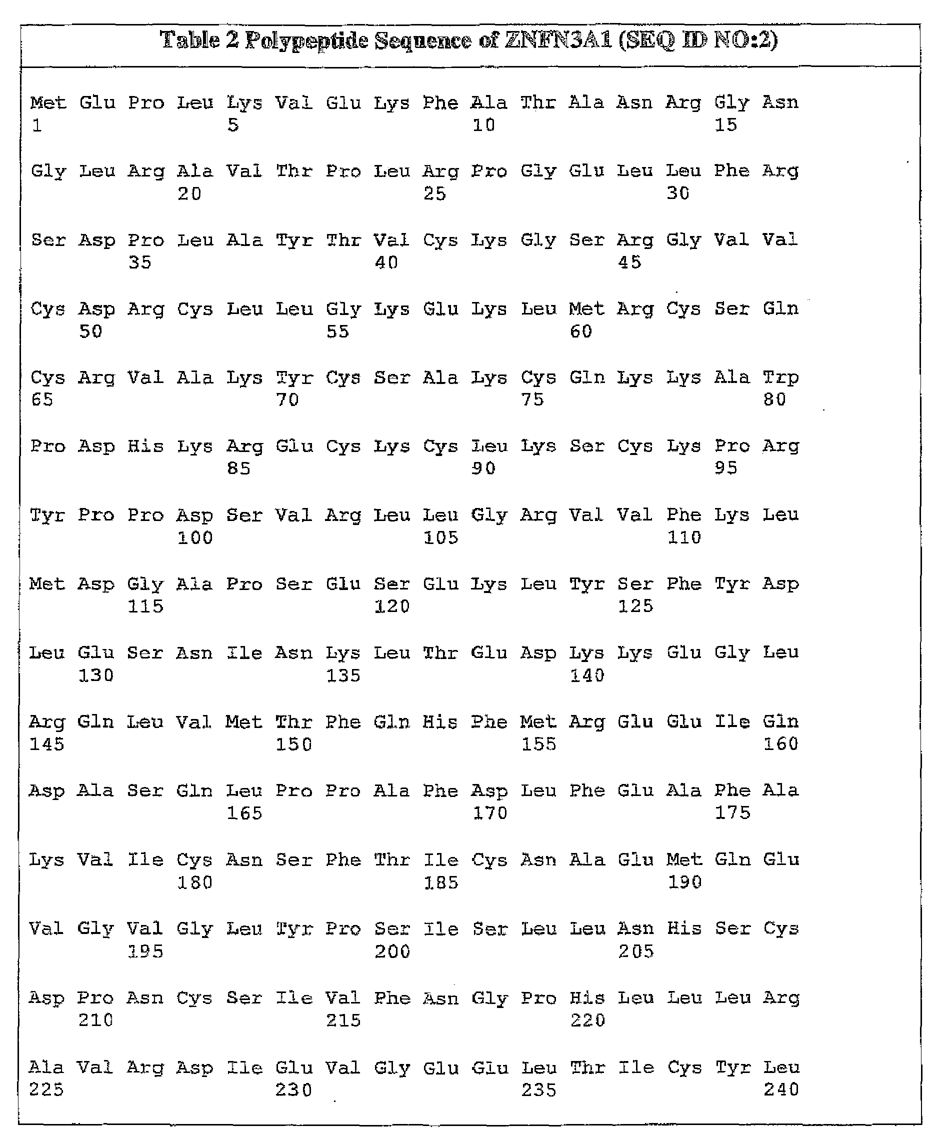

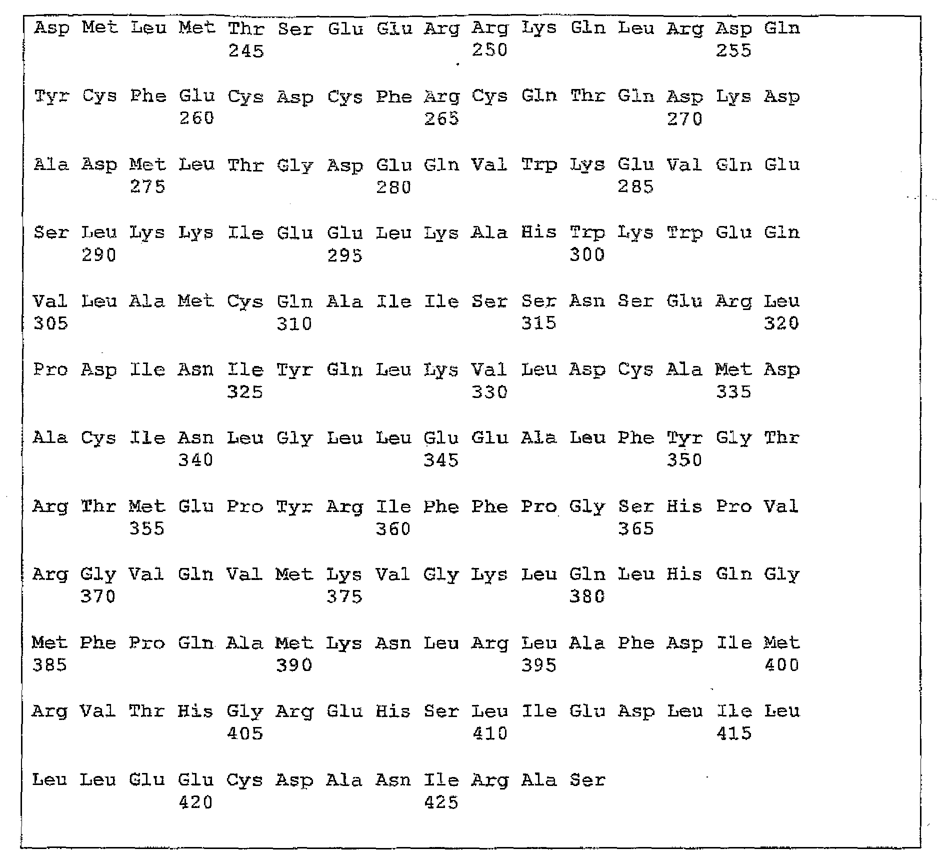

Exogenous expression of ZNFN3A1 in NTH3T3 cells resulted in increased cell growth. In contrast, suppression of its expression with antisense S-oligonucleotides resulted in a growth-inhibition of hepatoma cells.

### Methods of inhibiting cell growth

The present invention relates to inhibiting cell growth, *i.e,* cancer cell growth by inhibiting ZNFN3A1 expression. ZNFN3A1 expression is inhibited by small interfering RNA (siRNA) that specifically target of the ZNFN3A1 gene. A ZNFN3A1 target includes, for example, nucleotides 451-471, 532-552, 623-643, 625-645, 636-656, 726-746, 923-943, 1065-1085, and 1258-1278 of SEQ ID NO:1.

In non-mammalian cells, double-stranded RNA (dsRNA) has been shown to exert a strong and specific silencing effect on gene expression, which is referred as RNA interference (RNAi) (3). dsRNA is processed into 20-23 nucleotide dsRNA called small interfering RNA (siRNA) by an enzyme containing RNase III motif. The siRNA specifically targets complementary mRNA with a multicomponent nuclease complex (4, 5). In mammalian cells, siRNA composed of 20 or 21-mer dsRNA with 19 complementary nucleotides and 3' terminal noncomplementary dimmers of thymidine or uridine, have been shown to have a gene specific knock-down effect without inducing global changes in gene expression (6). In addiction, plasmids containing small nuclear RNA (snRNA) U6 or polymerise III H1-RNA promoter effectively produce such short RNA recruiting type III class of RNA polymerase III and thus can constitutively suppress its target mRNA (7, 8).

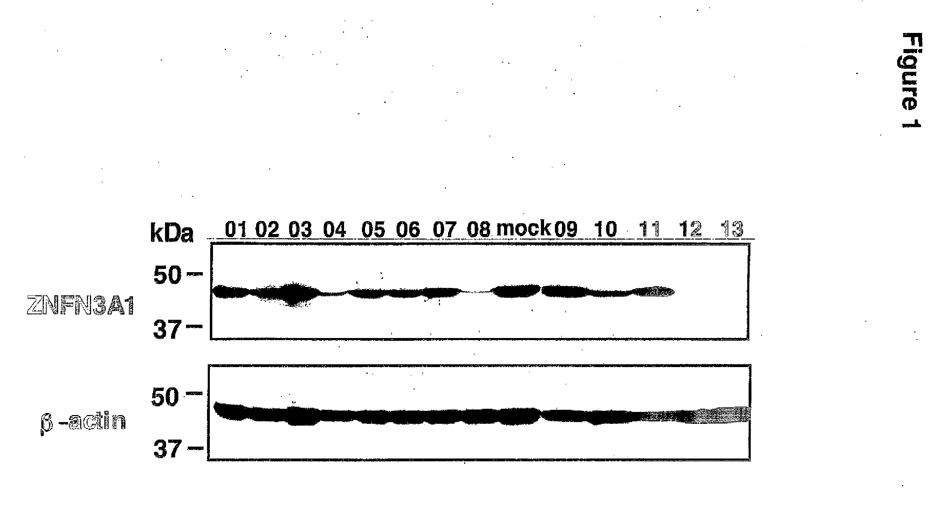

13 different expression plasmids were constructed to express hairpin-looped ZNFN3A1-siRNA (See Example 2). The plasmids were tested for their ability to inhibit cell growth. Four plasmids (psiU6BX-ZNFN3A1-4, -8, -12 and -13) markedly and five plasmids (psiU6BX-ZNFN3A1-2, -5, -6, -7, and -10) moderately suppressed endogeneous ZNFN3A1 expression, while the remaining four plasmids (psiU6EX-ZNFN3A1-1, -3, -9 and -11 exhibited no or little effect on the expression. (Figure 1). Various human hepatoma and colorectal cancer cells transfected with psiU6BX-siZNFN3A1-12, showed reduced number of surviving cells compared to control plasmids. FACTS analysis revealed that their death was due to apoptosis.

The growth of cells are inhibited by contacting a cell, with a composition containing a ZNFN3A1 siRNA. The cell is further contacted with a transfection agent. Suitable transfection agents are known in the art. By inhibition of cell growth is meant the cell proliferates at a lower rate or has decreased viability compared to a cell not exposed to the composition. Cell growth is measured by methods known in the art such as, the MTT cell proliferation assay.

The ZNFN3A1-siRNA is directed to a single target ZNFN3A1 gene sequence. Alternatively, the siRNA is directed to multiple target ZNFN3A1 gene sequences. For example, the composition contains ZNFN3A1- siRNA directed to two, three, four, or five or more ZNFN3A1 target sequences. By ZNFN3A1 target sequence is meant a nucleotide sequence that is identical to a portion of the ZNFN3A1 gene. The target sequence can include the 5' untranslated (UT) region, the open reading frame (ORF) or the 3' untranslated region of the human ZNFN3A1 gene. Alternatively, the siRNA is a nucleic acid sequence complementary to an upstream or downstream modulator of ZNFN3A1 gene expression. Examples of upstream and downstream modulators include, a transcription factor that binds the ZNFN3A1 gene promoter, a kinase or phosphatase that interacts with the ZNFN3A1 polypeptide, a ZNFN3A1 promoter or enhancer.

ZNFN3A1- siRNA which hybridize to target mRNA decrease or inhibit production of the ZNFN3A1 polypeptide product encoded by the ZNFN3A1 gene by associating with the normally single-stranded mRNA transcript, thereby interfering with translation and thus, expression of the protein. The siRNA is less than 500, 200, 100, 50, or 25 nucleotides in length. Preferably the siRNA is 19-25 nucleotides in length. Exemplary nucleic acid sequence for the production of ZNFN3A1-siRNA include the sequences of nucleotides 451-471 (SEQ ID NO:58), 532-552 (SEQ ID NO:60), 623-643 (SEQ ID NO:61), 625-645 (SEQ ID NO:62), 636-656 (SEQ ID NO:63), 726-746 (SEQ ID NO:64), 923-943 (SEQ ID NO:66), 1065-1085 (SEQ ID NO:69), or 1258-1278 (SEQ ID NO:69) of SEQ ID NO:1 as the target sequence Furthermore, in order to enhance the inhibition activity of the siRNA, nucleotide "u" can be added to 3' end of the antisense strand of the target sequence. The number of "u"s to be added is at least 2, generally 2 to 10, preferably 2 to 5. The added "u"s form single strand at the 3'end of the antisense strand of the siRNA.

The cell is any cell that expresses or over-expresses ZNFN3A1. The cell is a hepatic cell or an epithelial cell such as a colon cell. Alternatively, the cell is a tumor cell such as a carcinoma, adenocarcinoma, blastoma, leukemia, myeloma, or sarcoma. The cell is a hepatocellular carcinoma or a colorectal adenocarcinoma cell.

An ZNFN3A1 -siRNA is directly introduced into the cells in a form that is capable of binding to the mRNA transcripts. Alternatively, the DNA encoding the ZNFN3A1-siRNA is in a vector.

Vectors are produced for example by cloning a ZNFN3A1 target sequence into an expression vector operatively-linked regulatory sequences flanking the ZNFN3A1 sequence in a manner that allows for expression (by transcription of the DNA molecule) of both strands (Lee, N.S., Dohjima, T., Bauer, G., Li, H., Li, M.-J., Ehsani, A.,Salvaterra, P., and Rossi, J. (2002) Expression of small interfering RNAs targeted against HIV-1 rev transcripts in human cells. Nature Biotechnology 20: 500-505.). An RNA molecule that is antisense to ZNFN3A1 mRNA is transcribed by a first promoter (*e.g*., a promoter sequence 3' of the cloned DNA) and an RNA molecule that is the sense strand for the ZNFN3A1 mRNA is transcribed by a second promoter (*e.g*., a promoter sequence 5' of the cloned DNA). The sense and anti sense strands hybridize *in vivo* to generate siRNA constructs for silencing of the ZNFN3A1 gene. Alternatively, two constructs are utilized to create the sense and anti-sense strands of a siRNA construct. Cloned ZNFN3A1 can encode a construct having secondary structure, e.g., hairpins, wherein a single transcript has both the sense and complementary antisense sequences from the target gene.
A loop sequence consisting of an arbitrary nucleotide sequence can be located between the sense and antisense sequence in order to form the hairpin loop structure. Thus, the present invention also provides siRNA having the general formula 5'-[A]-[B]-[A']-3', wherein [A] is a ribonucleotide sequence corresponding to a sequence selected from the group consisting of nucleotides 451-471 (SEQ ID NO:58), 532-552 (SEQ ID NO:60), 623-643 (SEQ ID NO:61), 625-645 (SEQ ID NO:62), 636-656 (SEQ ID NO:63), 726-746 (SEQ ID NO:64), 923-943 (SEQ ID NO:66),1065-1085 (SEQ ID NO:68), and 1258-1278 (SEQ ID NO:69) of SEQ ID NO:1,
[B] is a ribonucleotide sequence consisting of 3 to 23 nucleotides, and
[A'] is a ribonucleotide sequence consisting of the complementary sequence of [A]

The region [A] hybridizes to [A'], and then a loop consisting of region [B] is formed. The loop sequence may be preferably 3 to 23 nucleotide in length. The loop sequence, for example, can be selected from group consisting of following sequences (http://www.ambion.com/techlib/tb/tb_506.html). Furthermore, loop sequence consisting of 23 nucleotides also provides active siRNA (Jacque, J.-M., Triques, K., and Stevenson, M. (2002) Modulation of HIV-1 replication by RNA interference. Nature 418 : 435-438.).

AUG :Sui, G., Soohoo, C., Affar, E.B., Gay, F., Shi, Y., Forrester, W.C., and Shi, Y. (2002) A DNA vector-based RNAi technology to suppress gene expression in mammalian cells. Proc. Natl. Acad. Sci. US A 99(8): 5515-5520.

CCC, CCACC or CCACACC: Paul, C.P., Good, P.D., Winer, I., and Engelke, D.R. (2002) Effective expression of small interfering RNA in human cells. Nature Biotechnology 20 : 505-508.

UUCG: Lee, N.S., Dohjima, T., Bauer, G., Li, H., Li, M.-J., Ehsani, A., Salvaterra, P., and Rossi, J. (2002) Expression of small interfering RNA targeted against HIV-1 rev transcripts in human cells. Nature Biotechnology 20 : 500-505.

CTCGAG or AAGCUU: Editors of Nature Cell Biology (2003) Whither RNAi? Nat Cell Biol. 5:489-490.

UUCAAGAGA: Yu, J.-Y., DeRuiter, S.L., and Turner, D.L. (2002) RNA interference by expression of short-interfering RNAs and hairpin RNAs in mammalian cells. Proc. Natl. Acad. Sci. USA 99(9): 6047-6052.

For example, preferable siRNAs having hairpin loop structure of the present invention are shown below. In the following structure, the loop sequence can be selected from group consisting of AUG, CCC, UUCG, CCACC, CTCGAG, AAGCUU, CCACACC, and UUCAAGAGA. Preferable loop sequence is UUCAAGAGA ("ttcaagaga" in DNA).
aaucagagaagcuuuacucau-[B]-augaguaaagcuucucugauu (for target sequence of SEQ ID NO:58)
aacucguaaugacauuucaac-[B]-guugaaaugucauuacgaguu (for target sequence of SEQ ID NO:60)
aaaagugaucugcaacncuuu-[B]-aaagaguugcagaucacuuuu (for target sequence of SEQ ID NO:61)
aagugaucugcaacucuuuca-[B]-ugaaagaguugcagaucacuu (for target sequence of SEQ ID NO:62)
aacucuuncaccaucuguaau-[B]-auuacagauggugaaagaguu (for target sequence of SEQ ID NO:63)
aacuguucgauuguguucaau-[B]-auugaacacaaucgaacaguu (for target sequence of SEQ ID NO:64)
aacuggugaugagcaaguaug-[B]-cauacuugcucaucaccaguu (for target sequence of SEQ ID NO:66)
aacaucuaccagcugaaggug-[B]-caccuucagcugguagauguu (for target sequence of SEQ ID NO:68)
aagcaaugaagaaucugagac-[B]-gucucagauucuucauugcuu (for target sequence of SEQ ID NO:69)

The regulatory sequences flanking the ZNFN3A1 sequence are identical or are different, such that their expression can be modulate independently, or in temporal or spatial manner. siRNAs are transcribed intracellularly by cloning the ZNFN3A1 gene templates into a vector containing, *e.g*., a RNA pol III transcription unit from the small nuclear RNA (snRNA) U6 or the human H1 RNA promoter. For introducing the vector into the cell, transfection-enhancing agent can be used. FuGENE (Rochediagnostices), Lipofectamin 2000 (Invitrogen), Oligofectamin (Invitrogen), and Nucleofactor (Wako pure Chemical) are useful as the transfection-enhancing agent.

Oligonucleotides and oligonucleotides complementary to various portions of ZNFN3A1 mRNA were tested *in vitro* for their ability to decrease production of ZNFN3A1 in tumor cells (e.g., using the Alexander and HepG2 hepatocellular carcinoma (HCC) cell line and the HCT116 and SW948 colorectal cancer cell line) according to standard methods. A reduction in ZNFN3A1 gene product in cells contacted with the candidate siRNA composition compared to cells cultured in the absence of the candidate composition is detected using ZNFN3A1-specific antibodies or other detection strategies. Sequences which decrease production of ZNFN3A1 in *in vitro* cell-based or cell-free assays are then tested for there inhibitory effects on cell growth. Sequences which inhibit cell growth in *in vitro* cell-based assay are test in *in vivo* in rats or mice to confirm decreased ZNFN3A1 production and decreased tumor cell growth in animals with malignant neoplasms.

### Methods of treating malignant tumors

Patients with tumors characterized as over-expressing ZNFN3A1 are treated by administering ZNFN3A1-siRNA. siRNA therapy is used to inhibit expression of ZNFN3A1 in patients suffering from or at risk of developing, for example, hepatocellular carcinomas, or colorectal cancer. Such patients are identified by standard methods of the particular tumor type. Hepatocellular carcinoma is diagnosed for example, by enlargement of the liver, tomography, ultrasound or biopsy. Colorectal cancer is diagnosed for example, by blood in stool, colonoscopy, flexible sigmoidoscopy, CEA Assay, double contrast barium enema CT Scan, tomography or biopsy.

Treatment is efficacious if the treatment leads to clinical benefit such as, a reduction in expression of ZNFN3A1, or a decrease in size, prevalence, or metastatic potential of the tumor in the subject. When treatment is applied prophylactically, "efficacious" means that the treatment retards or prevents tumors from forming or prevents or alleviates a symptom of clinical symptom of the tumor. Efficaciousness is determined in association with any known method for diagnosing or treating the particular tumor type.

siRNA therapy is carried out by administering to a patient siRNA by standard vectors and/or gene delivery systems. Suitable gene delivery systems may include liposomes, receptor-mediated delivery systems, or viral vectors such as herpes viruses, retroviruses, adenoviruses and adeno-associated viruses, among others. A reduction in ZNFN3A1 production results in a decrease ZNPN3A1 complex formation with KIAA0054 protein and DNA polymerase II or a decrease in ZNFN3A1 protein expression. A therapeutic nucleic acid composition is formulated in a pharmaceutically acceptable carrier. The therapeutic composition may also include a gene delivery system as described above. Pharmaceutically acceptable carriers are biologically compatible vehicles which are suitable for administration to an animal, *e.g*., physiological saline. A therapeutically effective amount of a compound is an amount which is capable of producing a medically desirable result such as reduced production of a ZNTN3A1 gene product, reduction of cell growth, *e.g.,* proliferation, or a reduction in tumor growth in a treated animal.

Parenteral administration, such as intravenous, subcutaneous, intramuscular, and intraperitoneal delivery routes, may be used to deliver ZNFN3A1-siRNA compositions. For treatment of hepatic tumors, direct infusion the portal vein is useful.

Dosages for any one patient depends upon many factors, including the patient's size, body surface area, age, the particular nucleic acid to be administered, sex, time and route of administration, general health, and other drugs being administered concurrently. Dosage for intravenous administration of nucleic acids is from approximately 10⁶ to 10²² copies of the polynucleotide.

The polynucleotides are administered by standard methods, such as by injection into the interstitial space of tissues such as muscles or skin, introduction into the circulation or into body cavities or by inhalation or insufflation. Polynucleotides are injected or otherwise delivered to the animal with a pharmaceutically acceptable liquid carrier, *e.g*., a liquid carrier, which is aqueous or partly aqueous. The polynucleotides are associated with a liposome (*e.g*., a cationic or anionic liposome). The polynucleotide includes genetic information necessary for expression by a target cell, such as a promoters.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting

### Brief Description of the Drawings

Fig. 1 is a photograph of an immunoblot showing the effect of ZNFN3A1 siRNAs on exogeneous ZNFN3A1 expression in COS7 cells.
Fig. 2 is a photograph of an immunoblot showing the expression of ZNFN3A1 protein in hepatoma and colon cancer cell lines.
Fig. 3 is a photograph of an immunoblot showing the effect of ZNFN3A1-siRNAs on endogeneous ZNFN3A1 expression in SNU475 cell transfected with psiU6BX-ZNFN3A1-1, -4, -12 or psiU6BX-mock plasmids.
Fig. 4A -B are bar charts showing the effect of ZNFN3A1-siRNAs on cell growth in SNU475 cells. Viability of transfected cells was measured by MTT assay 6 (Panel A) and 9 (panel B) days after the transfection.
Fig. 5 are bar charts showing growth suppressive effect of ZNFN3A1-siRNAs in various human hepatoma and colon cancer cells. Viability of transfected cells was measured by MTT assay, 9 to 12 days after the transfection.
Figure 6 is an illustration showing cell death in response to ZNFN3A1-siRNAs in SNU475 cell detected by FACS analysis.

### Best Mode for Carrying out the Invention

The invention will be further described in the following examples, which do not limit the scope of the invention described in the claims.

### [Example 1] General Methods

### Cell lines and tissue specimens

Human hepatoma cell lines Alexander and HepG2, human colon cancer lines HCT116 and SW948, and monkey fibroblast cell line COS7 were obtained from the American Type Culture Collection (ATCC). Human hepatoma cell line Huh7 was obtained from Japanese Collection of Research Bioresources (JCRB). Human hepatoma cell lines, SNU398, SNU423, SNU449 and SNU475 were obtained from the Korea cell-line bank. All these cells are publicly available.

All cell lines were grown in monolayers in appropriate medial Dulbecco's modified Eagle's medium for Alexander, Huh7, HepG2 and COS7; McCoy's 5A for HCT116; Leibovitz's L-15 for SW948; RPM11640 for SNU398, SNU423, SNU449 and SNU475 supplemented with 10% fetal bovine serum and 1% antibiotic/antimycotic solution (Sigma.). All cells were maintained at 37 °C in humid air with 5% CO₂, (Alexander, Huh7, HepG2, SNU398, SNU423, SNU449, SNU475, HCT116, and COS7) or without CO₂ (SW948).

### Cloning of ZNFN3A1

Cloning of *ZNFN3A1* was done by PCR using KOD-plus (TOYOBO). For E. Coli expression, coding region of *ZNFN3A1* was cloned in the *Eco*R I-*Kpn* I site of pET21a. For mammalian cell expression, coding region of *ZNFN3A1* was cloned in the *Eco*R I-K*pn* I site of pcDNA3.1 I (+) and (-) (Invitrogen), *Eco*R I*-Kpn* I site ot pFLAG and *Eco*R I*-Kpn* I site of pEGFP (Clontech). Coding region of *KIAA0054* was cloned in the *Eco*R I*-Xho* I site of pCMV-HA (Clontech).

### ZNFN3A1 Polyclonal Antibody Production

Rabbit anti-ZNFN3A1 polyclonal antibody was generated. Full coding sequence of *ZNFN3A1* was amplified by PCR reaction using testis cDNA as a template and cloned in pET21 a (Novagen). The cloned vector was transfected into BL21-CodonPlus® competent cells (Stratagene). Recombinant ZNFN3A1 protein was induced by 1.0 mM IPTG at 30°C for 6 h. His-ZNFN3A1 fusion protein was purified using Pro Bond™ Resin (Invitrogen). Rabbits were immunized ten times with purified His-ZNFN3A1. Immunoblotting with this polyclonal antibody showed single 50 kD band of FLAG-tagged ZNFN3A1, which was identical pattern to that detected using anti-FLAG monoclonal antibody (Sigma) (data not shown).

### RNA preparation and RT-PCR

Total RNA was extracted with Trizol reagent (Life technologies) according to the manufacturer's protocol. Ten-microgram aliquots of total RNA were reversely transcribed for single-stranded cDNAs using poly dT₁₂₋₁₈ primer (Amersham Biosciences) with Superscript II reverse transcriptase (Life Technologies). Each single-stranded cDNA was diluted for subsequent PCR amplification. Standard RT-PCR was carried out in a 20 µl volume of PCR buffer (TAKARA), and amplified for 4 min at 94 °C for denaturing, followed by 20 (for *GAPDH*) or 30 (for *ZNFN3A1*) cycles of 94 °C for 30 s, 56 °C for 30 s, 72 °C for 30 s, in the Gene Amp PCR system 9700 (Perkin-Elmer). Primer sequence were as follows,
for *GAPDH*
forward; 5'-ACAACAGCCTCAAGATCATCAG-3' (SEQ ID No: 29) and
reverse; 5'-GGTCCACCACTGACACGTTG-3' (SEQ ID No: 30),
for or *ZNFN3A1*
forward; 5'-TTCCCGATATCAACATCTACCAG-3' (SEQ ID No: 31) and
reverse; 5'-AGTGTGTGACCTCAATAAGGCAT-3' (SEQ ID No: 32).

### Construction of psiU6BX6 Plasmid

The DNA fragment encoding siRNA was inserted into the GAP at nucleotide 485-490 as indicated (-) in the following plasmid sequence (SEQ ID No: 33).

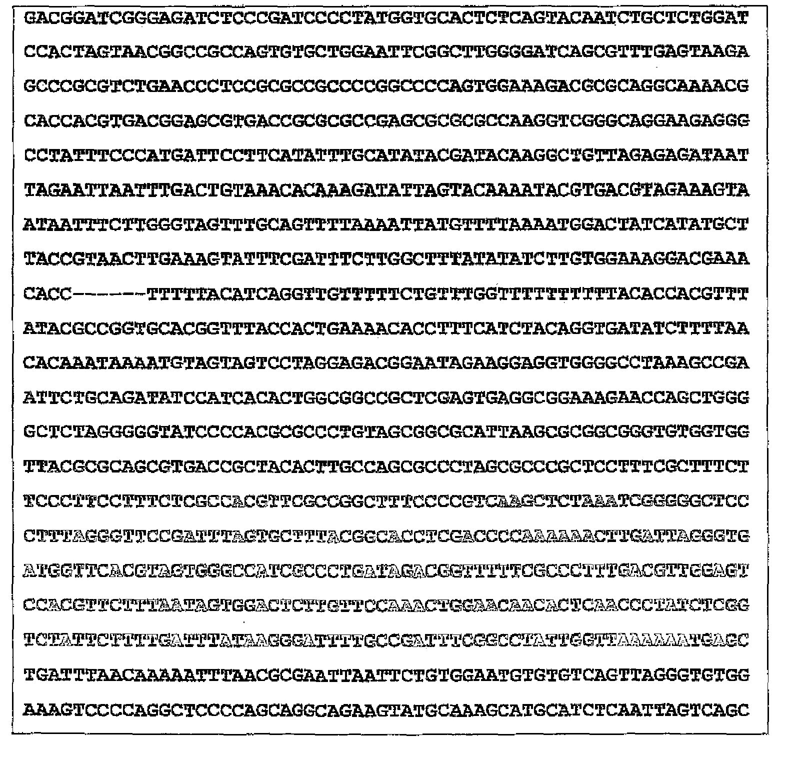

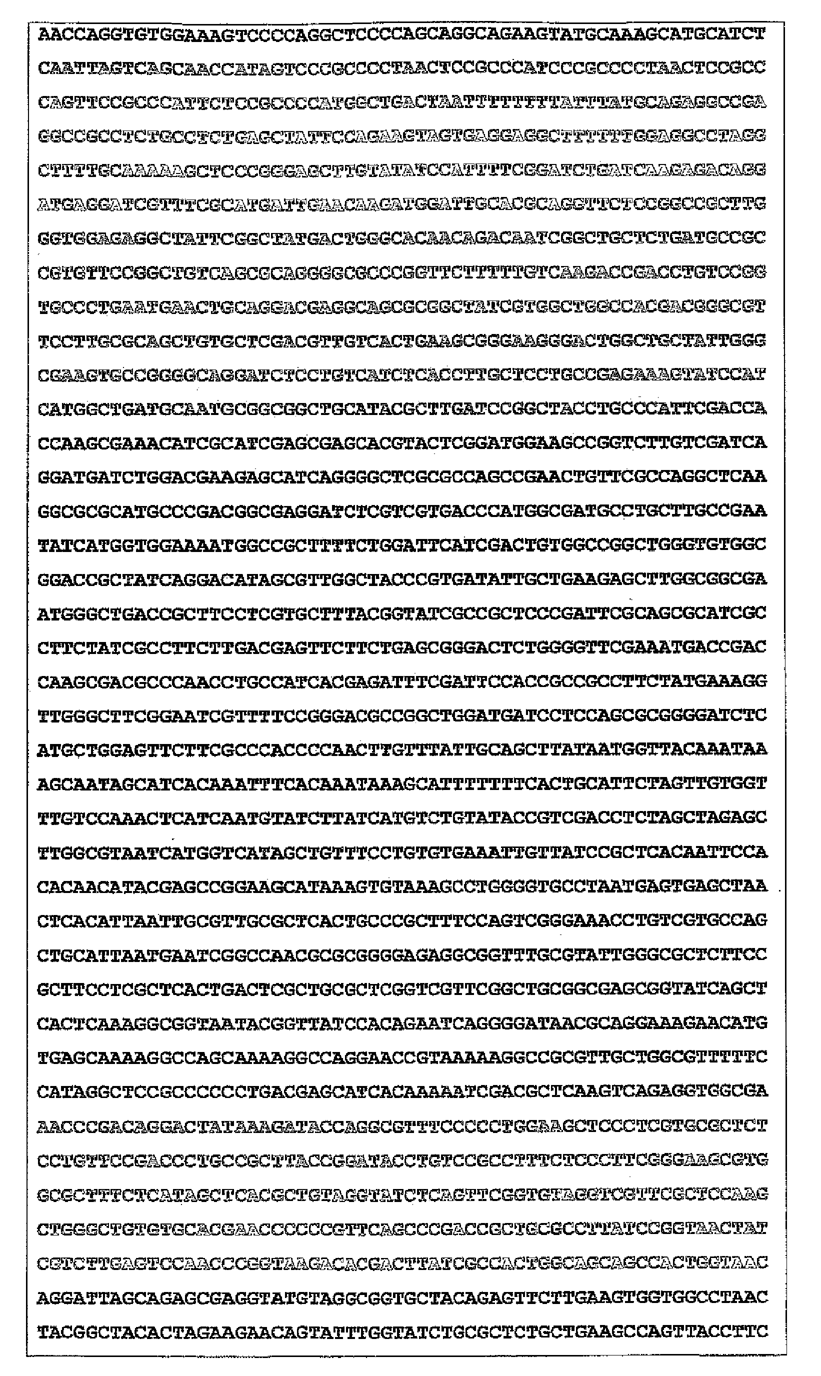

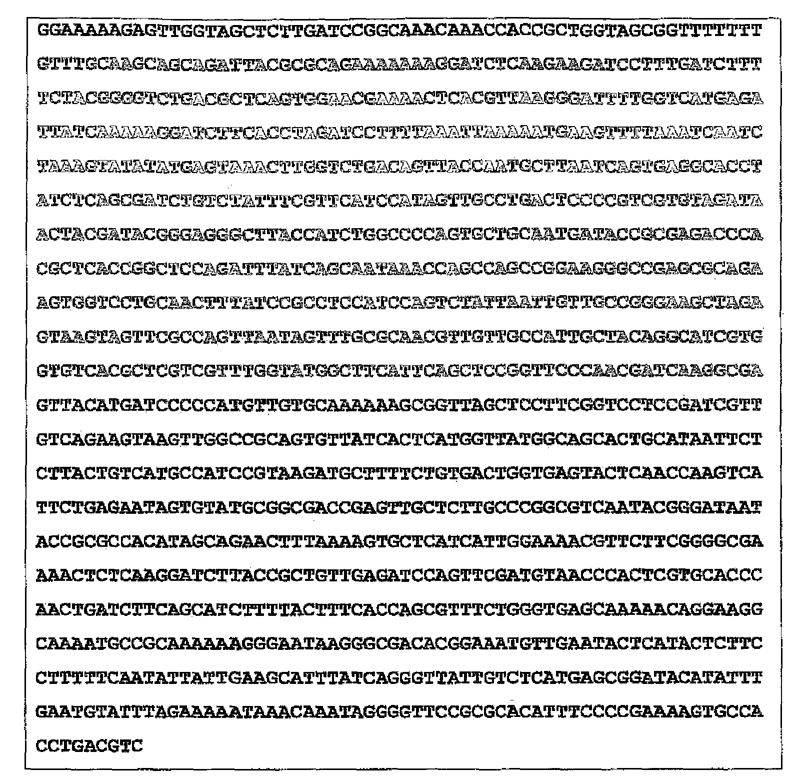

snRNA U6 gene is reported to be transcribed by RNA polymerase III, which produce short transcripts with uridines at the 3' end. The genomic fragment of the snRNA U6 gene containing the promoter region was amplified by PCR using a set of primers,
5'-GGGGATCAGCGTTTGAGTAA-3' (SEQ ID No: 34), and
5'-TAGGCCCCACCTCCTTCTAT-3' (SEQ ID No: 35) and human placental DNA as a template. The product was purified and cloned into pCR plasmid vector using a TA cloning kit according to the supplier's protocol (Invitrogen). The *Bam*HI, *Xho*I fragment containing the snRNA U6 gene was purified and cloned into nucleotide 1257 to 56 fragment of pcDNA3.1(+) plasmid, which was amplified by PCR with a set of primer, 5'-TGCGGATCCAGAGCAGATTGTACTGAGAGT-3' (SEQ ID No: 36) and 5'-CTCTATCTCGAGTGAGGCGGAAAGAACCA-3' (SEQ ID No: 37). The ligated DNA was used for a template of PCR with primers,
5'-TTTAAGCTTGAAGACTATTTTTACATCAGGTTGTTTTTCT-3' (SEQ ID No: 38) and
5'-TTTAAGCTTGAAGACACGGTGTTTCGTCCTTTCCACA-3' (SEQ ID No: 39). The product was digested with HindIII, which was subsequently self-ligated to produce psiU6BX vector plasmid. For the control, psiU6BX-EGFP was prepared by cloning double-stranded oligonucleotides of
5'- CACCGAAGCAGCACGACTTCTTCTTCAAGAGAGAAGAAGTCGTGCT GCTTC-3' (SEQ ID No: 40) and
5'- AAAAGAAGCAGCACGACTTCTTCTCTCTTGAAGAAGAAGTCGTGCT GCTTC -3' (SEQ ID No: 41) into the BbsI site in the psiU6BX vector.

### Immunoblotting

The polyclonal antibody to ZNFN3A1 was previously purified from sera of immunized rabbits with recombinant His-tagged ZNFN3A1 protein. Proteins were separated by 10% SDS-PAGE and immunoblotted with the anti-ZNFN3A1 antibody. HRP-conjugated goat anti-rabbit IgG (Santa Cruz Biotechnology, Santa Cruz, CA) served as the secondary antibody for the ECL Detection System (Amersham Pharmacia Biotech, Piscataway, NJ). Immunoblotting with the anti-ZNFN3A1 antibody showed single 50 kD band of FLAG-tagged ZNFN3A1, which was identical pattern to that detected using anti-FLAG antibody

### 3-(4,5-dimethylthiazol-2-yl)-2,5-diphenyltetrazolium bromide (MTT) assay

Cells were transfected with psiU6BX-siZNFN3A1 or control plamids and maintained in the culture media supplemented with optimum concentration of geneticin. Six to twelve days after transfection, the medium was replaced with fresh medium containing 500 µg/ml of MTT (3-(4,5-dimethylthiazol-2-yl)-2,5-diphenyl tetrazolium bromide) (Sigma) and the plates were incubated for four hours at 37°C. Subsequently, the cells were lysed by the addition of 1 ml of 0.01 N HCI/10%SDS and. absorbance of lysates was measured with an ELISA plate reader at a test wavelength of 570 nm (reference, 630 nm). The cell viability was represented by the absorbance compared to that of control cells.

### Flow cytometry

The effect of ZNFN3A1 in cell cycle progression was determined by flow cytometry. Cells were plated at a density of 1X10⁵ cells/100 mm dish. The cells were trypsinized at the given time course, collected in PBS and fixed in 70% cold ethanol. After RNase treatment, cells were stained with propidium iodide (50 µg/ml) in PBS. Flow cytometry was performed on a Becton Dickinson FACScan and analyzed by CellQuest and ModFit software (Verity Software House), The percentages of nuclei in GO/G1, S and G2M phases of the cell cycle, and any sub-G1 population were determined from at least 20,000 ungated cells.

To examine the role of ZNFN3A1-siRNAs in cell cycle, 1X10⁵ of SNU475 cells transfected with psiU6BX-ZNFN3A1 or control plasmids were collected by trypsinization at 5 days after transfection. After fixation in 70% cold ethanol, cells were treated with RNase and propidium iodide (50 µg/ml) in PBS, and analyzed by a FACScan (Becton Dickinson, San Jose, CA). The percentages of cells in G0/G1, S and G2/M phases of the cell cycle, and any sub-G1 population were determined from at least 20,000 ungated cells using ModFit software (Verity Software House)

### [Example 2] Production and Characterization of Plasmids Expressing ZNFN3A1 siRNAs

The entire coding sequence of *ZNFN3A1* was amplified with a set of primers, 5'-GGGGTACCAGGATGGAGCCGCTGAAGCTGG-3' (SEQ ID No: 42), and 5'-GGGAATTCTTAGGATGCTCTGATGTTGGCGTCG-3' (SEQ ID No: 43) and cloned into the appropriate cloning sites of pcDNA 3.1(+) vector (Invitrogen) (pcDNA-ZNFN3A1). Plasmids expressing ZNFN3A1-siRNAs were prepared by cloning of double-stranded oligonucleotides into psiU6BX vector.

The nucleotide sequence of the siRNAs were designed using an siRNA design computer program available from the Ambion website.
(http://www.ambion.com/techIib/misc/siRNA_5nder.html). Briefly, nucleotide sequences for siRNA synthesis are selected using the following protocol.

### Selection of siRNA Target Sites:

1. Starting with the AUG start codon of the ZNFN3A1 transcript, scan downstream for an AA dinucleotide sequences. The occurrence of each AA and the 3' adjacent 19 nucleotides are recorded as potential siRNA target sites. Tuschl et al. recommend against designing siRNA to the 5' and 3' untranslated regions (UTRs) and regions near the start codon (within 75bases) as these may be richer in regulatory protein binding sites. UTR-binding proteins and/or translation initiation complexes may interfere with binding of the siRNA endonuclease complex.
2. The potential target sites are compared to the appropriate genome database (human, mouse, rat, etc.) to eliminate target sequences with significant homology to other coding sequences.
3. Qualifying target sequences are selected for synthesis. Several target sequences along the length of the gene are selected for evaluation.
The oligonucleotides used for ZNFN3A1 siRNAs are shown below. psiU6BX-ZNFN3A1 1-13 (siRNA 1-13) were prepared by cloning the following double-stranded oligonucleotide into the Bbs1 site of the psiU6 vector. The corresponding nucleotide position relative to the ZNFN3A1 nucleic acid sequence of SEQ ID NO:1 is listed for each oligonucleotide sequence. Each oligionucleotide is a combination of a sense nucleotide sequence and an antisense nucleotide sequence of the target sequence ZNFN3A1. The nucleotide sequences of the hairpin loop structure and target sequence of siRNA1 to 13 are shown in SEQ ID NO:44 to SEQ ID NO:56 and SEQ ID NO:57 to SEQ ID NO:69, respectively (endonuclease recognition cites are eliminated from each hairpin loop structure sequence).
psiUSBX-ZNFN3A1-1 /siRNA1: (nucleotide numbers 426-446 of SEQ ID No: 1) 5'- CACCAAACTTATGGATGGAGCACCTTTCAAGAGAAGGTGCTCCATCCATAA GTTT-3' (SEQ ID NO: 3) and
5'- AAAAAAACTTATGGATGGAGCACCTTCTCTTGAAAGGTGCTCCATCCATAA GTTT-3' (SEQ ID NO: 4)
psiU6BX-ZNFN3A1-2 /siRNA2: (nucleotide numbers 451-471 of SEQ ID No: 1) 5'-CACCAATCAGAGAAGCTTTACTCATTTCAAGAGAATGAGTAAAGCTTCTCTG ATT-3' (SEQ ID NO: 5) and
5'-AAAAAATCAGAGAAGCTTTACTCATTCTCTTGAAATGAGTAAAGCTTCTCTG ATT-3' (SEQ ID NO: 6)
psiU6BX-ZNFN3A1-3 /siRNA3 : (nucleotide numbers 495-515 of SEQ ID No: 1)
siRNA2; 5'- CACCAACAAACTGACTGAAGATAAGTTCAAGAGACTTATCTTCAG TCAGTTTGTT-3' (SEQ ID NO: 7) and
5'-AAAAAACAAACTGACTGAAGATAAGTCTCTTGAACTTATCTT-CAGTCAGTTT GTT-3' (SEQ ID NO: 8)
psiU6BX-ZNFN3A1-4 /siRNA4: (nucleotide numbers 532-552 of SEQ ID No: 1) 5'- CACCAACTCGTAATGACATTTCAACTTCAAGAGAGTTGAAATGTCATTACG AGTT-3' (SEQ ID NO: 9)and
5'-AAAAAACTCGTAATGACATTTCAACTCTCTTGAAGTTGAAATGTCATTACGA GTT-3' (SEQ ID NO: 10)
psiU6BX-ZNFN3A1-5 /siRNA5: (nucleotide numbers 623-643 of SEQ ID No: 1) 5'- CACCAAAAGTGATCTGCAACTCTTTCAAGAGAAAAGAGTTGCAGATCAC TTTT-3' (SEQ ID NO: 11)and
5'-AAAAAAAAGTGATCTGCAACTCTTTTCTCTTGAAAAAGAGTTGCAGATCACT TTT-3' (SEQ ID NO: 12)
psiU6BX-ZNFN3A1-6 /siRNA6: (nucleotide numbers 625-645 of SEQ ID No: 1) 5'- CACCAAGTGATCTGCAACTCTTTCATTCAAGAGATGAAAGAGTTGCAGATC ACTT-3' (SEQ ID NO: 13)and
5'-AAAAAAGTGATCTGCAACTCTTTCATCTCTTGAATGAAAGAGTTGCAGATCA CTT-3' (SEQ ID NO: 14)
psiU6BX-ZNFN3A1-7 /siRNA7: (nucleotide numbers 636-656 of SEQ ID No: 1) 5'- CACCAACTCTTTCACCATCTGTAATTTCAAGAGAATTACAGATGGTGAAAG AGTT-3' (SEQ ID NO: 15)and
5'-AAAAAACTCTTTCACCATCTGTAATTCTCTTGAAATTACAGATGGTGAAAGA GTT-3' (SEQ ID NO: 16)
psiU6BX-ZNFN3A1-8 /siRNA8: (nucleotide numbers 726-746 of SEQ ID No: 1) 5'-CACCAACTGTTCGATTGTGTTCAATTTCAAGAGAATTGAACACAATCGAACA GTT-3' (SEQ ID NO: 17)and
5'-AAAAAACTGTTCGATTGTGTTCAATTCTCTTGAAATTGAACACAATCGAACA GTT-3' (SEQ ID NO: 18)
psiU6BX-ZNFN3A1-9 /siRNA9: (nucleotide numbers 906-926 of SEQ ID No: 1)
5'- CACCAAGGATGCTGATATGCTAACTTTCAAGAGAAGTTAGCATATCAGCAT CCTT-3' (SEQ ID NO: 19)and
5'AAAAAAGGATGCTGATATGCTAACTTCTCTTGAAAGTTAGCATATCAGCATC CTT-3' (SEQ ID NO: 20)
psiU6BX-ZNFN3A1-10 /siRNA10: (nucleotide numbers 923-943 of SEQ ID No: 1)
5'- CACCAACTGGTGATGAGCAAGTATGTTCAAGAGACATACTTGCTCATCACC AGTT-3' (SEQ ID NO: 21) and
5'-AAAAAACTGGTGATGAGCAAGTATGTCTCTTGAACATACTTGCTCATCACCA GTT-3' (SEX ID NO: 22)
psiU6BX-ZNFN3A1-11 /siRNA11: (nucleotide numbers 937-957 of SEQ ID No: 1)
5'- CACCAAGTATGGAAGGAAGTCAAGTTCAAGAGACTTGAACTTCCTTCCAT ACTT-3' (SEQ ID NO: 23)and
5'-AAAAAAGTATGGAAGGAAGTTCAAGTCTCTTGAACTTGAACTTCCTTCCATA CTT-3' (SEQ ID NO: 24)
psiU6BX-ZNFN3A1-12 /siRNA12: (nucleotide numbers 1065-1085 of SEQ ID No: 1)
5'-CACCAACATCTACCAGCTGAAGGTGTTCAAGAGACACCTTCAGCTGGTAGA TGTT-3' (SEQ ID NO: 25)and
5'-AAAAAACATCTACCAGCTGAAGGTGTCTCTTGAACACCTTCAGCTGGTAGAT GTT-3' (SEQ ID NO: 26)
psiU6BX-ZNFN3A1-13 /siRNA13: (nucleotide numbers 1258-1278 of SEQ ID No: 1)
5'- CACCAAGCAATGAAGAATCTGAGACTTCAAGAGAGTCTCAGATTCTTCATT GCTT-3' (SEQ ID NO: 27) and
5'-AAAAAAGCAATGAAGAATCTGAGACTCTCTTGAAGTCTCAGATTCTTCATTG CTT-3' (SEQ ID NO: 28)

psiU6BX-siZNFN3A1 or psiU6BX-mock plasmids were transfected with pcDNA-ZNFN3A1 into COS7 cells using FuGENE6 reagent according to the supplier's recommendations (Roche). The plasmids were solely transfected into SNU479 cells expressing abundant amount of endogeneous ZNFN3A1. Whole extracts of the cells were lysed 2 days after the transfection and utilized for immunoblot analysis.

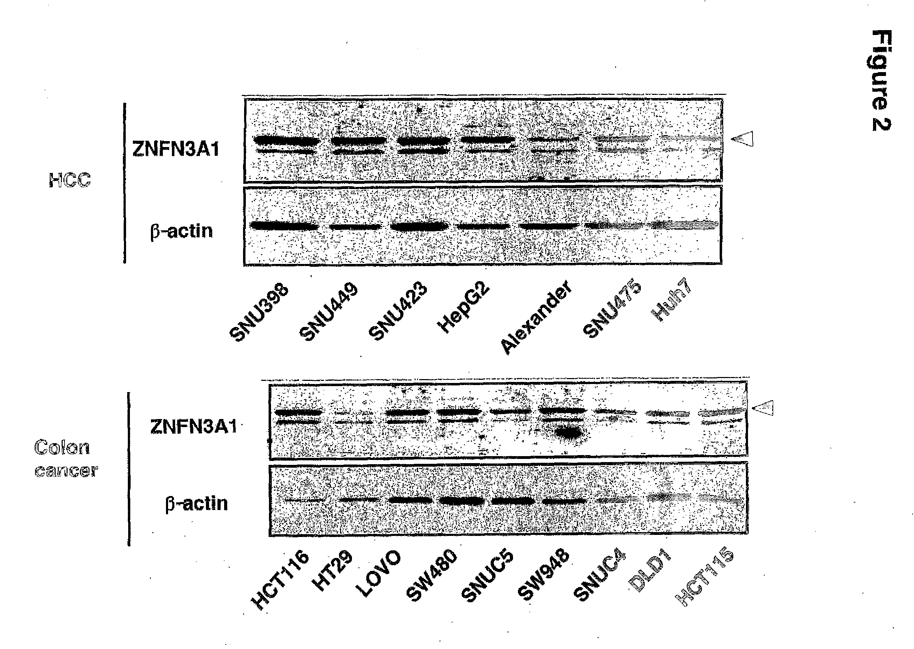

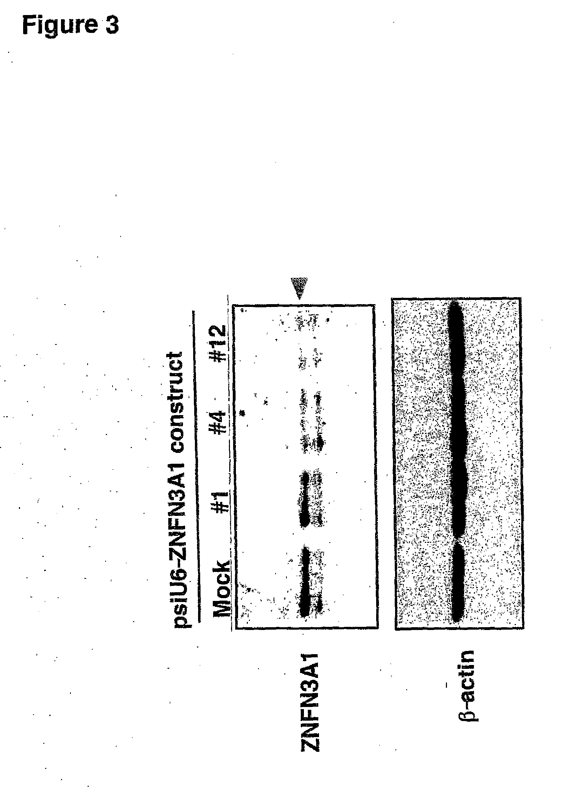

Among the 13 different expression plasmids expressing ZNFN3A1 siRNAs, psiU6BX-ZNFN3A1-8, -12, and -13 most significantly reduced expression of exogeneous ZNFN3A1 by western blot analysis, when they were transfected into COS7 cells together with pcDNA-ZNFN3A1. Among other plasmids, psiU6BX-ZNFN3A1-4 showed marked reduction, and psiU6BX-ZNFN3A1-2, -5, -6, -7 and -10 exerted moderate suppression, whereas psiU6BX-ZNFN3A1-1, -3, -9 and -11 had no or little effect on the expression (Figure 1). To further examine RNAi activity of ZNFN3A1 siRNAs, we transected psiU6BX-ZNFN3A1-1, -4, -12, or psiU6BX-mock into SNU475 cells that express abundant amount of ZNFN3A1 (Figure 2). Western blot analysis using the extracts of transfected cells demonstrated marked reduction of endogeneous ZNFN3A1 by psiU6BX-ZNFN3A1-12, and moderate suppression by psiU6BX-ZNFN3A1-4 compared to cells transfected with psiU6BX-mock. On the other hand transfection with psiU6BX-ZNFN3A1-1 did not affect expression of ZNFN3A1 (Figure 3).

### [Example 3] Growth suppression of hepatoma and colon cancer cells by ZNFN3A1 siRNA

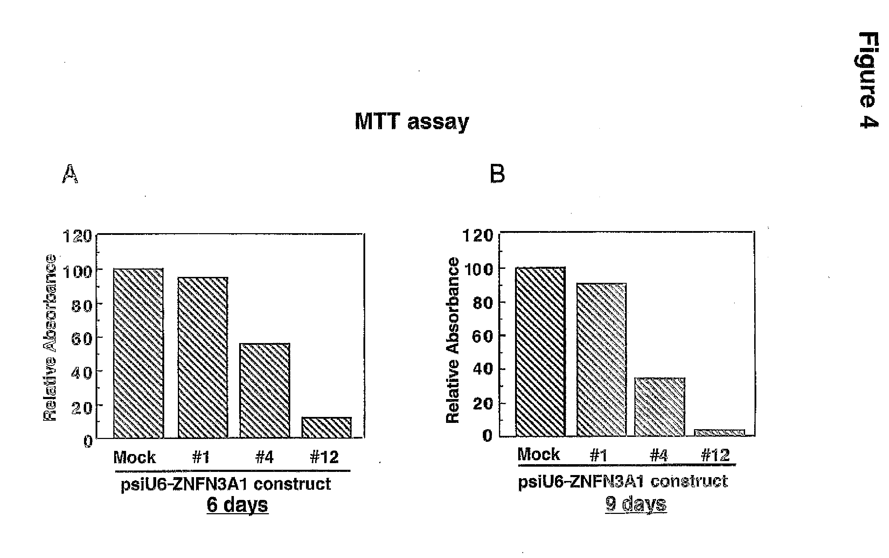

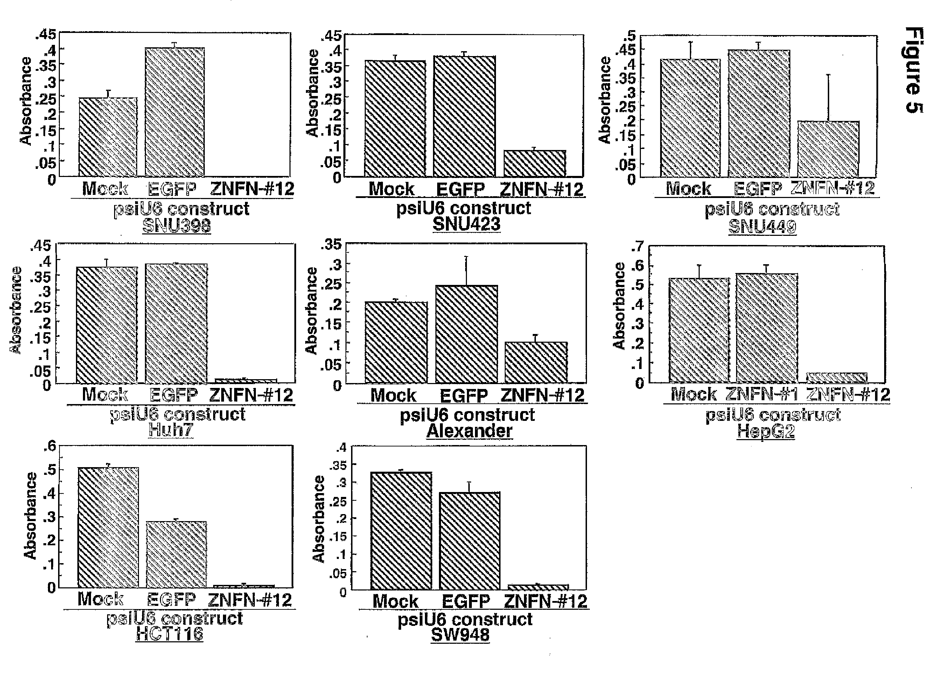

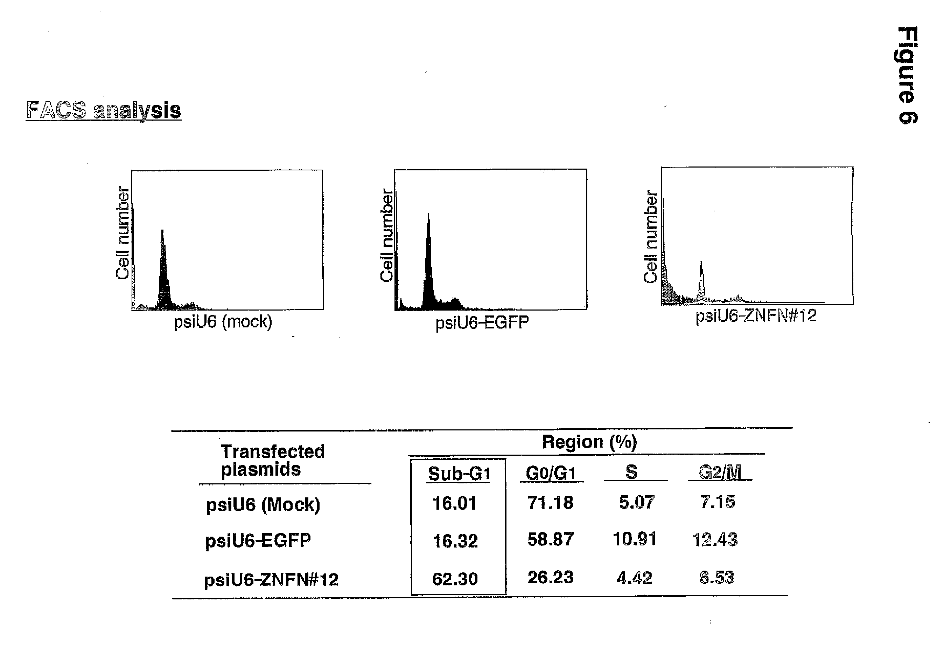

To test whether suppression of *ZNFN3A1* may result in growth suppression of hepatoma cells, SNU475 cells were transfected with either psiU6BX-ZNFN3A1-12, the vector that demonstrated the most knock down effect on the expression; psiU6BX-ZNFN3A1-4 which demonstrated mild silencing effect; psiU6BX-ZNFN3A1-1 which demonstrated no silencing effect, or psiU6BX-mock. MTT assays at both 6 days and 9 days of transfection showed that psiU6BX-ZNFN3A1-12 has the highest growth inhibitory effect and that psiU6BX-ZNFN3A1-1 did not change the number of surviving cells compared with cells transfected with psiU6EX-mock (Figure 4). The growth inhibitory effect of the plasmids was correlated to their gene silencing activity. To further demonstrate the growth inhibitory effect of ZNFN3A1-siRNAs, psiU6BX-ZNFN3A1-12; psiU6$X-EGFI' For the control, psiU6BX-EGFP was prepared by cloning the following double-stranded oligonucleotide
5'- CACCGAAGCAGCACGACTTCTTCTTCAAGAGAGAAGAAGTCGTGCT GCTTC-3' (SEQ ID No: 40) and
5'- AAAAGAAGCAGCACGACTTCTTCTCTCTTGAAGAAGAAGTCGTGCT GCTTC -3' (SEQ ID No: 41) into the BbsI site of the psiU6BX vector.
or psiU6BX-mosk was transfected into various hepatoma cell lines including SNU398, SNU423, SNU449, Huh7, Alexander, and HepG2 and two colon cancer cell lines, SW948 and HCT116. Transfection of psiU6BX-ZNFN3A1-12 significantly reduced number of surviving cells compared with that of psiU6BX-EGFP or psiU6BX-mock (Figure 5). Furthermore, FACS analysis demonstrated that transfection of psiU6BX-ZNFN3A1-12 increased the number of cells in sub-G1 phase (Figure 6). These results indicate that ZNFN3A1 contributes to aberrant cell growth and/or survival in a wide range of human cancer cells.

### Industrial Applicability

The present inventors have shown that the cell growth is suppressed by small interfering RNA (siRNA) that specifically target the ZNFN3A1 gene. Thus, this novel siRNAs are useful target for the development of anti-cancer pharmaceutical. For example, agents that block the expression of ZNFN3A1 or prevent its activity may find therapeutic utility as anti-cancer agents, particularly anti-cancer agents for the treatment of liver cancer or colon cancer, such as HCC or colorectal adenocarcinoma.

While the invention has been described in detail and with reference to specific embodiments thereof, it will be apparent to one skilled in the art that various changes and modifications can be made therein without departing from the spirit and scope of the invention.

### References

(1) Akriviadis EA, Llovet JM, Efremidis SC, Shouval D, Canelo R, Rings B, Meyers WC. Hepatocellular carcinoma. Br J Surg. 1998 Oct;85(10):1319-31.
(2) Okabe H, Satoh S, Kato T, Kitahara O, Yanagawa R, Yamaoka Y, Tsunoda T, Furukawa Y, Nakamura Y. Genome-wide analysis of gene expression in human hepatocellular carcinomas using cDNA microarray: identification of genes involved in viral carcinogenesis and tumor progression. Cancer Res. 2001 Mar 1;61(5):2129-37.
(3) Sharp PA. RNAi and double-strand RNA. Genes Dev. 1999 Jan 15;13(2):139-41..
(4) Hammond SM, Bernstein E, Beach D, Hannon GJ. An RNA-directed nuclease mediates post-transcriptional gene silencing in Drosophila cells. Nature. 2000 Mar 16;404(6775):293-6.
(5) Hannon GJ. RNA interference. Nature. 2002 Jul 11;418(6894):244-51.
(6) Elbashir SM, Harborth J, Lendeckel W, Yalcin A, Weber K, Tuschl T. Duplexes of 21-nucleotide RNAs mediate RNA interference in cultured mammalian cells. Nature. 2001 May 24;411(6836):494-8.
(7) Miyagishi M, Taira K. U6 promoter-driven siRNAs with four uridine 3' overhangs efficiently suppress targeted gene expression in mammalian cells.Nat Biotechnol. 2002 May;20(5):497-500
(8) Brummelkamp TR, Bernards R, Agami R. A System for Stable Expression of Short Interfering RNAs in Mammalian Cells Science. 296(5567):550-553, April 19, 2002.

Furthermore, the present invention relates to the following items:
1. A method of inhibiting tumor cell growth in a subject, comprising administering to said subject a composition comprising a ZNFN3A1 small interfering RNA (siRNA).
2. The method of item. 1, wherein said siRNA comprises a sense ZNFN3A1 nucleic acid and an anti-sense ZNFN3A1 nucleic acid.
3. The method of item 1, wherein said tumor cell is a colorectal cancer cell or liver cancer cell.
4. The method of item, 3, wherein the colorectal cancer cell is an adenocarcinoma cell.
5. The method of item 3, wherein the liver cancer cell is a hepatocellular carcinoma cell.
6. The method of item 2, said siRNA is specific for a ZNFN3A1 target selected from the group consisting of nucleotides 451-471, 532-552, 623-643, 625-645, 636-656,726-746, 923-943, 1065-1085, and 1258-1278 of SEQ ID NO:1.
7. The method of item 6, said siRNA has the general formula 5'-[A]-[B]-[A']-3',
   wherein [A] is a ribonucleotide sequence coresponding to a sequence selected from the group consisting of nucleotides 451-471,532-552,623-643,625-645,636-656,726-746,923-943,1065-1085, and 1258-1278 of SEQ ID NO:1,
   [B] is a ribonucleotide sequence consisting of 3 to 23 nucleotides, and
   [A'] is a ribonucleotide sequence consisting of the complementary sequence of [A].
8. The method of item 1, wherein said composition comprises a transfection-enhancing agent.
9. An isolated polynucleotide comprising a combination of a sense strand nucleic acid and an antisense strand nucleic acid, wherein said sense strand nucleic acid comprises nucleotide sequence selected from the group consisting of nucleotides 451-471, 532-552, 623-643, 625-645, 636-656,726-746,923-943, 1065-1085, and 1258-1278 of SEQ ID NO:1, and said antisense strand nucleic acid consists of complementary sequence thereof, respectivery.
10. The isolated polynucleotide of item 9, wherein said sense strand nucleic acid and antisense strand nucleic acid are on the same strand.
11. The isolated polynucleotide of item 9, wherein said sense strand nucleic acid consists of a nucleotide sequence shorter than about 100 nucleotides.
12. The isolated polynucleotides of, item 11, wherein said sense strand nucleic acid is shorter than about 75 nucleotides.
13. The isolated polynucleotides of item 12, wherein said sense strand nucleic acid is shorter than about 50 nucleotides.
14. The isolated polynucleotide of item 13. wherein said sense strand nucleic acid is shorter than about 25 nucleotides.
15. The isolated polynucleotide of item 14, wherein said sense strand nucleic acid is between about 19 and about 25 nucleotides in length.
16. A vector comprising a polynucleotide comprising a combination of a sense strand nucleic acid and an antisense strand nucleic acid, wherein said sense strand nucleic acid comprises nucleotide sequence selected from the group consisting of nucleotides 451-471, 532-552, 623-643, 625-645,636-656,726-746, 923-943, 1065-1085, and 1258-1278 of SEQ ID NO:1, and said antisense strand nucleic acid consists of complementary sequence thereof, respectivery.
17. The vector of item 16, wherein said polynucleotide has the general formula 5'-[A]-[B]-[A']-3', wherein [A] is a nucleotide sequence selected from the group consisting of nucleotides 451-471, 532-552, 623-643, 625-645, 636-656,726-746, 923-943,1065-1085, and 1258-1278 of SEQ ID NO:1,
   [B] is a nucleotide sequence consisting of 3 to 23 nucleotides, and
   [A'] is a nucleotide sequence consisting of the complementary sequence of [A].
18. A composition comprising at least one siRNA comprising a combination of a sense strand nuclei acid and an anti sense strand nucleic acid, wherein said sense strand nucleic acid comprises ribonucleotide sequence coresponding to a sequence selected from the group consisting of nucleotides 451-471, 532-552, 623-643, 625-645,636-656,726-746,923-943,1065-1085, and 1258-1278 of SEQ ID NO:1, and said antisense strand sequence consists of complementary sequence thereof, respectivery.
19. A double-stranded molecule comprising a sense strand and an antisense strand, wherein the sense strand comprises a ribonucleotide sequence corresponding to a ZNFN3A1 target sequence, and wherein the antisense strand comprises a ribonucleotide sequence which is complementary to said sense strand, wherein said sense strand and said antisense strand hybridize to each other to form said double-stranded molecule, and wherein said double-stranded molecule, when introduced into a cell expressing the ZNFN3A1 gene, inhibits expression of said gene.
20. The double-stranded molecule of item 19, wherein said ZNFN3A1 target sequence comprises at least about 10 contiguous nucleotides from SEQ ID No:1.
21. The double-stranded molecule of item 20, wherein said ZNFN3A1 target sequence comprises from about 1.9 to about 25 contiguous nucleotides from SEQ ID No:1.
22. The double-stranded molecule of item 21, wherein said ZNFN3A1 target sequence is selected from the group consisting of nucleotides 451-471,532-552, 623-643,625-645,636-656,726-746,923-943,1065-10S5, and 1258-1278 of SEQ ID NO:1.
23. The double-stranded molecule of item 19, wherein a single ribonucleotide transcript comprises the sense strand and the antisense strand, said double-stranded molecule further comprising a single-stranded ribonucleotide sequence linking said sense strand and said antisense strand.
24. The double-stranded molecule of item 19, wherein the double stranded molecule is an oligonucleotide of less than about 100 nucleotides in length.
25. The double-stranded molecule of item 24, wherein the double stranded molecule is an oligonucleotide of less than about 75 nucleotides in length.
26. The double-stranded molecule of item 25, wherein the double stranded molecule is an oligonucleotide of less than about 50 nucleotides in length.
27. The double-stranded molecule of item 26, wherein the double stranded molecule is an oligonucleotide of less than about 25 nucleotides in length.
28. The double-stranded polynucleotide of item 27, wherein the double stranded molecule is an oligonucleotide of between about 19 and about 25 nucleotides in length.
29. A vector encoding the double-stranded molecule of item 19.
30. The vector of item 29, wherein the vector encodes a transcript having a secondary structure, wherein the transcript comprises the sense strand and the antisense strand.
31. The vector of item 30, wherein the transcript further comprises a single-stranded ribonucleotide sequence linking said sense strand and said antisense strand.
32. A method to inhibit expression of the ZNFN3A1 gene in a cell of a biological sample, the method comprising introduction of a ribonucleic acid (RNA) into the cell in an amount sufficient to inhibit expression of the ZNFN3A1 gene, wherein the RNA is a double-stranded molecule comprising a sense strand and a antisense strand, wherein the sense strand comprises a ribonucleotide sequence corresponding to a ZNFN3A1 target sequence, and wherein the antisense strand comprises a ribonucleotide sequence which is complementary to said sense strand, wherein the sense and the antisense ribonucleotide strands hybridize to each other to form said double-stranded molecule, and wherein said double-stranded molecule, when introduced into a cell expressing the ZNFN3A1 gene, inhibits expression of said gene.

## Claims

1. A ZNFN3A1 small interfering RNA (siRNA) for use in inhibiting tumor cell growth in a subject.

2. The small interfering RNA of claim 1, wherein said siRNA comprises a sense ZNFN3A1 nucleic acid and an anti-sense ZNFN3A1 nucleic acid.

3. The small interfering RNA of claim 1, wherein said tumor cell is a colorectal cancer cell or liver cancer cell.

4. The small interfering RNA of claim 3, wherein the colorectal cancer cell is an adenocarcinoma cell.

5. The small interfering RNA of claim 3, wherein the liver cancer cell is a hepatocellular carcinoma cell.

6. The small interfering RNA of claim 2, said siRNA is specific for a ZNFN3A1 target selected from the group consisting of nucleotides 1065-1085, 451-471, 532-552, 623-643, 625-645, 636-656,726-746, 923-943, and 1258-1278 of SEQ ID NO:1.

7. The small interfering RNA of claim 6, said siRNA has the general formula 5'-[A]-[B]-[A']-3', wherein [A] is a ribonucleotide sequence coresponding to a sequence selected from the group consisting of nucleotides 1065-1085, 451-471, 532-552, 623-643, 625-645, 636-656,726-746, 923-943, and 1258-1278 of SEQ ID NO:1,
[B] is a ribonucleotide sequence consisting of 3 to 23 nucleotides, and
[A'] is a ribonucleotide sequence consisting of the complementary sequence of [A].

8. A vector comprising a polynucleotide comprising a combination of a sense strand nucleic acid and an antisense strand nucleic acid, wherein said sense strand nucleic acid comprises nucleotide sequence selected from the group consisting of nucleotides 1065-1085, 451-471, 532-552, 623-643, 625-645, 636-656,726-746, 923-943, and 1258-1278 of SEQ ID NO:1, and said antisense strand nucleic acid consists of complementary sequence thereof, respectivery.

9. The vector of claim 8, wherein said polynucleotide has the general formula 5'-[A]-[B]-[A']-3', wherein [A] is a nucleotide sequence selected from the group consisting of nucleotides 1065-1085, 451-471, 532-552, 623-643, 625-645, 636-656,726-746, 923-943, and 1258-1278 of SEQ ID NO:1,
[B] is a nucleotide sequence consisting of 3 to 23 nucleotides, and
[A'] is a nucleotide sequence consisting of the complementary sequence of [A].

10. A composition comprising at least one siRNA comprising a combination of a sense strand nucleic acid and an antisense strand nucleic acid, wherein said sense strand nucleic acid comprises ribonucleotide sequence coresponding to a sequence selected from the group consisting of nucleotides 1065-1085, 451-471, 532-552, 623-643, 625-645, 636-656,726-746, 923-943, and 1258-1278 of SEQ ID NO:1, and said antisense strand sequence consists of complementary sequence thereof, respectivery.

11. A double-stranded molecule comprising a sense strand and an antisense strand, wherein the sense strand comprises a ribonucleotide sequence corresponding to a ZNFN3A1 target sequence, and wherein the antisense strand comprises a ribonucleotide sequence which is complementary to said sense strand, wherein said sense strand and said antisense strand hybridize to each other to form said double-stranded molecule, and wherein said double-stranded molecule, when introduced into a cell expressing the ZNFN3A1 gene, inhibits expression of said gene.

12. The double-stranded molecule of claim 11, wherein said ZNFN3A1 target sequence comprises at least about 10 contiguous nucleotides from SEQ ID No:1.

13. The double-stranded molecule of claim 12, wherein said ZNFN3A1 target sequence comprises from about 19 to about 25 contiguous nucleotides from SEQ ID No:1.

14. The double-stranded molecule of claim 13, wherein said ZNFN3A1 target sequence is selected from the group consisting of nucleotides 1065-1085, 451-471, 532-552, 623-643, 625-645, 636-656,726-746, 923-943, and 1258-1278 of SEQ ID NO:1.

15. The double-stranded molecule of claim 11, wherein a single ribonucleotide transcript comprises the sense strand and the antisense strand, said double-stranded molecule further comprising a single-stranded ribonucleotide sequence linking said sense strand and said antisense strand.

16. The double-stranded molecule of claim 11, wherein the double stranded molecule is an oligonucleotide of between about 19 and about 25 nucleotides in length.

17. A vector encoding the double-stranded molecule of claim 11.

18. The vector of claim 17, wherein the vector encodes a transcript having a secondary structure, wherein the transcript comprises the sense strand and the antisense strand.

19. The vector of claim 18, wherein the transcript further comprises a single-stranded ribonucleotide sequence linking said sense strand and said antisense strand.


SEQUENCE LISTING

<110> ONCOTHERAPY SCIENCE, INC.
JAPAN AS REPRESENTED BY THE PRESIDENT OF THE UNIVERSITY OF TOKYO

<120> COMPOSITIONS AND METHODS OF INHIBITING CELL GROWTH

<130> ONC-A0301P

<150> US 60/450,644
<151> 2003-02-28

<160> 69

<170> PatentIn version 3.1

<210> 1
<211> 1622
<212> DNA
<213> Homo sapiens

<220>
<221> CDS
<222> (96)..(1382)
<223>

<400> 1
gtgcgcgcag ggcgcaggcg cgcgggtccc ggcagcccgt gagacgcccg ctgctggacg 60

cgggtagccg tctgaggtgc cggagctgcg ggagg atg gag ccg ctg aag gtg 113
Met Glu Pro Leu Lys Val
1               5

gaa aag ttc gca acc gcc aac agg gga aac ggg ctg cgc gcc gtg acc 161
Glu Lys Phe Ala Thr Ala Asn Arg Gly Asn Gly Leu Arg Ala Val Thr
            10                  15                  20

ccg ctg cgc ccc gga gag cta ctc ttc cgc tcg gat ccc ttg gcg tac 209
Pro Leu Arg Pro Gly Glu Leu Leu Phe Arg Ser Asp Pro Leu Ala Tyr
        25                  30                  35

acg gtg tgc aag ggg agt cgt ggc gtc gtc tgc gac cgc tgc ctt ctc 257
Thr Val Cys Lys Gly Ser Arg Gly Val Val Cys Asp Arg Cys Leu Leu

40                    45                    50

ggg aag gaa aag ctg atg cga tgc tct cag tgc cgc gtc gcc aaa tac          305
Gly Lys Glu Lys Leu Met Arg Cys Ser Gln Cys Arg Val Ala Lys Tyr
55                  60                  65                  70

tgt agt gct aag tgt cag aaa aaa gct tgg cca gac cac aag cgg gaa          353
Cys Ser Ala Lys Cys Gln Lys Lys Ala Trp Pro Asp His Lys Arg Glu
                75                  80                  85

tgc aaa tgc ctt aaa agc tgc aaa ccc aga tat cct cca gac tcc gtt          401
Cys Lys Cys Leu Lys Ser Cys Lys Pro Arg Tyr Pro Pro Asp Ser Val
            90                  95                  100

cga ctt ctt ggc aga gtt gtc ttc aaa ctt atg gat gga gca cct tca          449
Arg Leu Leu Gly Arg Val Val Phe Lys Leu Met Asp Gly Ala Pro Ser
        105                 110                 115

gaa tca gag aag ctt tac tca ttt tat gat ctg gag tca aat att aac          497
Glu Ser Glu Lys Leu Tyr Ser Phe Tyr Asp Leu Glu Ser Asn Ile Asn
    120                 125                 130

aaa ctg act gaa gat aag aaa gag ggc ctc agg caa ctc gta atg aca          545
Lys Leu Thr Glu Asp Lys Lys Glu Gly Leu Arg Gln Leu Val Met Thr
135                 140                 145                 150

ttt caa cat ttc atg aga gaa gaa ata cag gat gcc tct cag ctg cca          593
Phe Gln His Phe Met Arg Glu Glu Ile Gln Asp Ala Ser Gln Leu Pro
                155                 160                 165

cct gcc ttt gac ctt ttt gaa gcc ttt gca aaa gtg atc tgc aac tct          641
Pro Ala Phe Asp Leu Phe Glu Ala Phe Ala Lys Val Ile Cys Asn Ser
            170                 175                 180

ttc acc atc tgt aat gcg gag atg cag gaa gtt ggt gtt ggc cta tat          689
Phe Thr Ile Cys Asn Ala Glu Met Gln Glu Val Gly Val Gly Leu Tyr
        185                 190                 195

ccc agt atc tct ttg ctc aat cac agc tgt gac ccc aac tgt tcg att          737
Pro Ser Ile Ser Leu Leu Asn His Ser Cys Asp Pro Asn Cys Ser Ile
    200                 205                 210

gtg ttc aat ggg ccc cac ctc tta ctg cga gca gtc cga gac atc gag          785

Val Phe Asn Gly Pro His Leu Leu Leu Arg Ala Val Arg Asp Ile Glu
215                 220                 225                 230

gtg gga gag gag ctc acc atc tgc tac ctg gat atg ctg atg acc agt 833
Val Gly Glu Glu Leu Thr Ile Cys Tyr Leu Asp Met Leu Met Thr Ser
                235                 240                 245

gag gag cgc cgg aag cag ctg agg gac cag tac tgc ttt gaa tgt gac 881
Glu Glu Arg Arg Lys Gln Leu Arg Asp Gln Tyr Cys Phe Glu Cys Asp
            250                 255                 260

tgt ttc cgt tgc caa acc cag gac aag gat gct gat atg cta act ggt 929
Cys Phe Arg Cys Gln Thr Gln Asp Lys Asp Ala Asp Met Leu Thr Gly
        265                 270                 275

gat gag caa gta tgg aag gaa gtt caa gaa tcc ctg aaa aaa att gaa 977
Asp Glu Gln Val Trp Lys Glu Val Gln Glu Ser Leu Lys Lys Ile Glu
    280                 285                 290

gaa ctg aag gca cac tgg aag tgg gag cag gtt ctg gcc atg tgc cag 1025
Glu Leu Lys Ala His Trp Lys Trp Glu Gln Val Leu Ala Met Cys Gln
295                 300                 305                 310

gcg atc ata agc agc aat tct gaa cgg ctt ccc gat atc aac atc tac 1073
Ala Ile Ile Ser Ser Asn Ser Glu Arg Leu Pro Asp Ile Asn Ile Tyr
                315                 320                 325

cag ctg aag gtg ctc gac tgc gcc atg gat gcc tgc atc aac ctc ggc 1121
Gln Leu Lys Val Leu Asp Cys Ala Met Asp Ala Cys Ile Asn Leu Gly
            330                 335                 340

ctg ttg gag gaa gcc ttg ttc tat ggt act cgg acc atg gag cca tac 1169
Leu Leu Glu Glu Ala Leu Phe Tyr Gly Thr Arg Thr Met Glu Pro Tyr
        345                 350                 355

agg att ttt ttc cca gga agc cat ccc gtc aga ggg gtt caa gtg atg 1217
Arg Ile Phe Phe Pro Gly Ser His Pro Val Arg Gly Val Gln Val Met
    360                 365                 370

aaa gtt ggc aaa ctg cag cta cat caa ggc atg ttt ccc caa gca atg 1265
Lys Val Gly Lys Leu Gln Leu His Gln Gly Met Phe Pro Gln Ala Met
375                 380                 385                 390

aag aat ctg aga ctg gct ttt gat att atg aga gtg aca cat ggc aga     1313
Lys Asn Leu Arg Leu Ala Phe Asp Ile Met Arg Val Thr His Gly Arg
                395                 400                 405

gaa cac agc ctg att gaa gat ttg att cta ctt tta gaa gaa tgc gac     1361
Glu His Ser Leu Ile Glu Asp Leu Ile Leu Leu Leu Glu Glu Cys Asp
            410                 415                 420

gcc aac atc aga gca tcc taa gggaacgcag tcagagggaa atacggcgtg     1412
Ala Asn Ile Arg Ala Ser
        425

tgtctttgtt gaatgcctta ttgaggtcac acactctatg ctttgttagc tgtgtgaacc     1472
tctcttattg gaaattctgt tccgtgtttg tgtaggtaaa taaaggcaga catggtttgc     1532
aaaccacaag aatcattagt tgtagagaag cacgattata ataaattcaa aacatttggt     1592
tgaggatgcc aaaaaaaaaa aaaaaaaaaa                                      1622

<210>  2
<211>  428
<212>  PRT
<213>  Homo sapiens

<400>  2
Met Glu Pro Leu Lys Val Glu Lys Phe Ala Thr Ala Asn Arg Gly Asn
1               5                   10                  15

Gly Leu Arg Ala Val Thr Pro Leu Arg Pro Gly Glu Leu Leu Phe Arg
            20                  25                  30

Ser Asp Pro Leu Ala Tyr Thr Val Cys Lys Gly Ser Arg Gly Val Val
        35                  40                  45

Cys Asp Arg Cys Leu Leu Gly Lys Glu Lys Leu Met Arg Cys Ser Gln
    50                  55                  60

Cys Arg Val Ala Lys Tyr Cys Ser Ala Lys Cys Gln Lys Lys Ala Trp
65                  70                  75                  80

Pro Asp His Lys Arg Glu Cys Lys Cys Leu Lys Ser Cys Lys Pro Arg
                85                  90                  95

Tyr Pro Pro Asp Ser Val Arg Leu Leu Gly Arg Val Val Phe Lys Leu

            100                 105                 110

Met Asp Gly Ala Pro Ser Glu Ser Glu Lys Leu Tyr Ser Phe Tyr Asp
        115                 120                 125

Leu Glu Ser Asn Ile Asn Lys Leu Thr Glu Asp Lys Lys Glu Gly Leu
    130                 135                 140

Arg Gln Leu Val Met Thr Phe Gln His Phe Met Arg Glu Glu Ile Gln
145                 150                 155                 160

Asp Ala Ser Gln Leu Pro Pro Ala Phe Asp Leu Phe Glu Ala Phe Ala
                165                 170                 175

Lys Val Ile Cys Asn Ser Phe Thr Ile Cys Asn Ala Glu Met Gln Glu
            180                 185                 190

Val Gly Val Gly Leu Tyr Pro Ser Ile Ser Leu Leu Asn His Ser Cys
        195                 200                 205

Asp Pro Asn Cys Ser Ile Val Phe Asn Gly Pro His Leu Leu Leu Arg
    210                 215                 220

Ala Val Arg Asp Ile Glu Val Gly Glu Glu Leu Thr Ile Cys Tyr Leu
225                 230                 235                 240

Asp Met Leu Met Thr Ser Glu Glu Arg Arg Lys Gln Leu Arg Asp Gln
                245                 250                 255

Tyr Cys Phe Glu Cys Asp Cys Phe Arg Cys Gln Thr Gln Asp Lys Asp
            260                 265                 270

Ala Asp Met Leu Thr Gly Asp Glu Gln Val Trp Lys Glu Val Gln Glu
        275                 280                 285

Ser Leu Lys Lys Ile Glu Glu Leu Lys Ala His Trp Lys Trp Glu Gln
    290                 295                 300

Val Leu Ala Met Cys Gln Ala Ile Ile Ser Ser Asn Ser Glu Arg Leu
305                 310                 315                 320

Pro Asp Ile Asn Ile Tyr Gln Leu Lys Val Leu Asp Cys Ala Met Asp
                325                 330                 335

Ala Cys Ile Asn Leu Gly Leu Leu Glu Glu Ala Leu Phe Tyr Gly Thr
            340                 345                 350

Arg Thr Met Glu Pro Tyr Arg Ile Phe Phe Pro Gly Ser His Pro Val
        355                 360                 365

Arg Gly Val Gln Val Met Lys Val Gly Lys Leu Gln Leu His Gln Gly
    370                 375                 380

Met Phe Pro Gln Ala Met Lys Asn Leu Arg Leu Ala Phe Asp Ile Met
385                 390                 395                 400

Arg Val Thr His Gly Arg Glu His Ser Leu Ile Glu Asp Leu Ile Leu
                405                 410                 415

Leu Leu Glu Glu Cys Asp Ala Asn Ile Arg Ala Ser
            420                 425

<210>  3
<211>  55
<212>  DNA
<213>  Artificial

<220>
<223>  An artificially synthesized origonucleotide sequence for siRNA

<400>  3
caccaaactt atggatggag cacctttcaa gagaaggtgc tccatccata agttt          55

<210>  4
<211>  55
<212>  DNA
<213>  Artificial

<220>
<223>  An artificially synthesized origonucleotide sequence for siRNA

<400>  4
aaaaaaactt atggatggag caccttctct tgaaaggtgc tccatccata agttt          55

<210> 5
<211> 55
<212> DNA
<213> Artificial

<220>
<223> An artificially synthesized origonucleotide sequence for siRNA

<400> 5
caccaatcag agaagcttta ctcatttcaa gagaatgagt aaagcttctc tgatt 55


<210> 6
<211> 55
<212> DNA
<213> Artificial

<220>
<223> An artificially synthesized origonucleotide sequence for siRNA

<400> 6
aaaaaatcag agaagcttta ctcattctct tgaaatgagt aaagcttctc tgatt 55


<210> 7
<211> 55
<212> DNA
<213> Artificial

<220>
<223> An artificially synthesized origonucleotide sequence for siRNA

<400> 7
caccaacaaa ctgactgaag ataagttcaa gagacttatc ttcagtcagt ttgtt 55


<210> 8
<211> 55
<212> DNA
<213> Artificial

<220>

<223>  An artificially synthesized origonucleotide sequence for siRNA

<400>  8
aaaaaacaaa ctgactgaag ataagtctct tgaacttatc ttcagtcagt ttgtt          55


<210>  9
<211>  55
<212>  DNA
<213>  Artificial

<220>
<223>  An artificially synthesized origonucleotide sequence for siRNA

<400>  9
caccaactcg taatgacatt tcaacttcaa gagagttgaa atgtcattac gagtt          55


<210>  10
<211>  55
<212>  DNA
<213>  Artificial

<220>
<223>  An artificially synthesized origonucleotide sequence for siRNA

<400>  10
aaaaaactcg taatgacatt tcaactctct tgaagttgaa atgtcattac gagtt          55


<210>  11
<211>  55
<212>  DNA
<213>  Artificial

<220>
<223>  An artificially synthesized origonucleotide sequence for siRNA

<400>  11
caccaaaagt gatctgcaac tctttttcaa gagaaaagag ttgcagatca ctttt          55


<210>  12

<211>  55
<212>  DNA
<213>  Artificial

<220>
<223>  An artificially synthesized origonucleotide sequence for siRNA

<400>  12
aaaaaaaagt gatctgcaac tcttttctct tgaaaaagag ttgcagatca ctttt          55

<210>  13
<211>  55
<212>  DNA
<213>  Artificial

<220>
<223>  An artificially synthesized origonucleotide sequence for siRNA

<400>  13
caccaagtga tctgcaactc tttcattcaa gagatgaaag agttgcagat cactt          55

<210>  14
<211>  55
<212>  DNA
<213>  Artificial

<220>
<223>  An artificially synthesized origonucleotide sequence for siRNA

<400>  14
aaaaaagtga tctgcaactc tttcatctct tgaatgaaag agttgcagat cactt          55

<210>  15
<211>  55
<212>  DNA
<213>  Artificial

<220>
<223>  An artificially synthesized origonucleotide sequence for siRNA

<400> 15
caccaactct ttcaccatct gtaatttcaa gagaattaca gatggtgaaa gagtt 55

<210> 16
<211> 55
<212> DNA
<213> Artificial

<220>
<223> An artificially synthesized origonucleotide sequence for siRNA

<400> 16
aaaaaactct ttcaccatct gtaattctct tgaaattaca gatggtgaaa gagtt 55

<210> 17
<211> 55
<212> DNA
<213> Artificial

<220>
<223> An artificially synthesized origonucleotide sequence for siRNA

<400> 17
caccaactgt tcgattgtgt tcaatttcaa gagaattgaa cacaatcgaa cagtt 55

<210> 18
<211> 55
<212> DNA
<213> Artificial

<220>
<223> An artificially synthesized origonucleotide sequence for siRNA

<400> 18
aaaaaactgt tcgattgtgt tcaattctct tgaaattgaa cacaatcgaa cagtt 55

<210> 19
<211> 55
<212> DNA

<213>  Artificial

<220>
<223>  An artificially synthesized origonucleotide sequence for siRNA

<400>  19
caccaaggat gctgatatgc taactttcaa gagaagttag catatcagca tcctt          55


<210>  20
<211>  55
<212>  DNA
<213>  Artificial

<220>
<223>  An artificially synthesized origonucleotide sequence for siRNA

<400>  20
aaaaaaggat gctgatatgc taacttctct tgaaagttag catatcagca tcctt          55


<210>  21
<211>  55
<212>  DNA
<213>  Artificial

<220>
<223>  An artificially synthesized origonucleotide sequence for siRNA

<400>  21
caccaactgg tgatgagcaa gtatgttcaa gagacatact tgctcatcac cagtt          55


<210>  22
<211>  55
<212>  DNA
<213>  Artificial

<220>
<223>  An artificially synthesized origonucleotide sequence for siRNA

<400>  22
aaaaaactgg tgatgagcaa gtatgtctct tgaacatact tgctcatcac cagtt          55

<210> 23
<211> 55
<212> DNA
<213> Artificial

<220>
<223> An artificially synthesized origonucleotide sequence for siRNA

<400> 23
caccaagtat ggaaggaagt tcaagttcaa gagacttgaa cttccttcca tactt 55


<210> 24
<211> 55
<212> DNA
<213> Artificial

<220>
<223> An artificially synthesized origonucleotide sequence for siRNA

<400> 24
aaaaaagtat ggaaggaagt tcaagtctct tgaacttgaa cttccttcca tactt 55


<210> 25
<211> 55
<212> DNA
<213> Artificial

<220>
<223> An artificially synthesized origonucleotide sequence for siRNA

<400> 25
caccaacatc taccagctga aggtgttcaa gagacacctt cagctggtag atgtt 55


<210> 26
<211> 55
<212> DNA
<213> Artificial

<220>
<223>  An artificially synthesized origonucleotide sequence for siRNA

<400>  26
aaaaaacatc taccagctga aggtgtctct tgaacacctt cagctggtag atgtt          55


<210>  27
<211>  55
<212>  DNA
<213>  Artificial

<220>
<223>  An artificially synthesized origonucleotide sequence for siRNA

<400>  27
caccaagcaa tgaagaatct gagacttcaa gagagtctca gattcttcat tgctt          55


<210>  28
<211>  55
<212>  DNA
<213>  Artificial

<220>
<223>  An artificially synthesized origonucleotide sequence for siRNA

<400>  28
aaaaaagcaa tgaagaatct gagactctct tgaagtctca gattcttcat tgctt          55


<210>  29
<211>  22
<212>  DNA
<213>  Artificial

<220>
<223>  An artificially synthesized primer sequence

<400>  29
acaacagcct caagatcatc ag                                              22

<210> 30
<211> 20
<212> DNA
<213> Artificial

<220>
<223> An artificially synthesized primer sequence

<400> 30
ggtccaccac tgacacgttg 20

<210> 31
<211> 23
<212> DNA
<213> Artificial

<220>
<223> An artificially synthesized primer sequence

<400> 31
ttcccgatat caacatctac cag 23

<210> 32
<211> 23
<212> DNA
<213> Artificial

<220>
<223> An artificially synthesized primer sequence

<400> 32
agtgtgtgac ctcaataagg cat 23

<210> 33
<211> 4869
<212> DNA
<213> Artificial

<220>
<223> An artificially synthesized plasmid sequence

<220>
<221> misc_feature
<222> (485)..(490)
<223> "n" indicates GAP.

<400> 33
gacggatcgg gagatctccc gatcccctat ggtgcactct cagtacaatc tgctctggat 60
ccactagtaa cggccgccag tgtgctggaa ttcggcttgg ggatcagcgt ttgagtaaga 120
gcccgcgtct gaaccctccg cgccgccccg gccccagtgg aaagacgcgc aggcaaaacg 180
caccacgtga cggagcgtga ccgcgcgccg agcgcgcgcc aaggtcgggc aggaagaggg 240
cctatttccc atgattcctt catatttgca tatacgatac aaggctgtta gagagataat 300
tagaattaat ttgactgtaa acacaaagat attagtacaa aatacgtgac gtagaaagta 360
ataatttctt gggtagtttg cagttttaaa attatgtttt aaaatggact atcatatgct 420
taccgtaact tgaaagtatt tcgatttctt ggctttatat atcttgtgga aaggacgaaa 480
caccnnnnnn tttttacatc aggttgtttt tctgtttggt ttttttttta caccacgttt 540
atacgccggt gcacggttta ccactgaaaa cacctttcat ctacaggtga tatcttttaa 600
cacaaataaa atgtagtagt cctaggagac ggaatagaag gaggtggggc ctaaagccga 660
attctgcaga tatccatcac actggcggcc gctcgagtga ggcggaaaga accagctggg 720
gctctagggg gtatccccac gcgccctgta gcggcgcatt aagcgcggcg ggtgtggtgg 780
ttacgcgcag cgtgaccgct acacttgcca gcgccctagc gcccgctcct ttcgctttct 840
tcccttcctt tctcgccacg ttcgccggct ttccccgtca agctctaaat cgggggctcc 900
ctttagggtt ccgatttagt gctttacggc acctcgaccc caaaaaactt gattagggtg 960
atggttcacg tagtgggcca tcgccctgat agacggtttt tcgccctttg acgttggagt 1020
ccacgttctt taatagtgga ctcttgttcc aaactggaac aacactcaac cctatctcgg 1080
tctattcttt tgatttataa gggattttgc cgatttcggc ctattggtta aaaaatgagc 1140
tgatttaaca aaaatttaac gcgaattaat tctgtggaat gtgtgtcagt tagggtgtgg 1200
aaagtcccca ggctccccag caggcagaag tatgcaaagc atgcatctca attagtcagc 1260
aaccaggtgt ggaaagtccc caggctcccc agcaggcaga agtatgcaaa gcatgcatct 1320
caattagtca gcaaccatag tcccgcccct aactccgccc atcccgcccc taactccgcc 1380
cagttccgcc cattctccgc cccatggctg actaattttt tttatttatg cagaggccga 1440
ggccgcctct gcctctgagc tattccagaa gtagtgagga ggcttttttg gaggcctagg 1500
cttttgcaaa aagctcccgg gagcttgtat atccattttc ggatctgatc aagagacagg 1560
atgaggatcg tttcgcatga ttgaacaaga tggattgcac gcaggttctc cggccgcttg 1620
ggtggagagg ctattcggct atgactgggc acaacagaca atcggctgct ctgatgccgc 1680
cgtgttccgg ctgtcagcgc aggggcgccc ggttcttttt gtcaagaccg acctgtccgg 1740
tgccctgaat gaactgcagg acgaggcagc gcggctatcg tggctggcca cgacgggcgt 1800
tccttgcgca gctgtgctcg acgttgtcac tgaagcggga agggactggc tgctattggg 1860
cgaagtgccg gggcaggatc tcctgtcatc tcaccttgct cctgccgaga aagtatccat 1920
catggctgat gcaatgcggc ggctgcatac gcttgatccg gctacctgcc cattcgacca 1980
ccaagcgaaa catcgcatcg agcgagcacg tactcggatg gaagccggtc ttgtcgatca 2040
ggatgatctg gacgaagagc atcaggggct cgcgccagcc gaactgttcg ccaggctcaa 2100
ggcgcgcatg cccgacggcg aggatctcgt cgtgacccat ggcgatgcct gcttgccgaa 2160

tatcatggtg gaaaatggcc gcttttctgg attcatcgac tgtggccggc tgggtgtggc 2220
ggaccgctat caggacatag cgttggctac ccgtgatatt gctgaagagc ttggcggcga 2280
atgggctgac cgcttcctcg tgctttacgg tatcgccgct cccgattcgc agcgcatcgc 2340
cttctatcgc cttcttgacg agttcttctg agcgggactc tggggttcga aatgaccgac 2400
caagcgacgc ccaacctgcc atcacgagat ttcgattcca ccgccgcctt ctatgaaagg 2460
ttgggcttcg gaatcgtttt ccgggacgcc ggctggatga tcctccagcg cggggatctc 2520
atgctggagt tcttcgccca ccccaacttg tttattgcag cttataatgg ttacaaataa 2580
agcaatagca tcacaaattt cacaaataaa gcattttttt cactgcattc tagttgtggt 2640
ttgtccaaac tcatcaatgt atcttatcat gtctgtatac cgtcgacctc tagctagagc 2700
ttggcgtaat catggtcata gctgtttcct gtgtgaaatt gttatccgct cacaattcca 2760
cacaacatac gagccggaag cataaagtgt aaagcctggg gtgcctaatg agtgagctaa 2820
ctcacattaa ttgcgttgcg ctcactgccc gctttccagt cgggaaacct gtcgtgccag 2880
ctgcattaat gaatcggcca acgcgcgggg agaggcggtt tgcgtattgg gcgctcttcc 2940
gcttcctcgc tcactgactc gctgcgctcg gtcgttcggc tgcggcgagc ggtatcagct 3000
cactcaaagg cggtaatacg gttatccaca gaatcagggg ataacgcagg aaagaacatg 3060
tgagcaaaag gccagcaaaa ggccaggaac cgtaaaaagg ccgcgttgct ggcgtttttc 3120
cataggctcc gcccccctga cgagcatcac aaaaatcgac gctcaagtca gaggtggcga 3180
aacccgacag gactataaag ataccaggcg tttccccctg gaagctccct cgtgcgctct 3240
cctgttccga ccctgccgct taccggatac ctgtccgcct ttctcccttc gggaagcgtg 3300
gcgctttctc atagctcacg ctgtaggtat ctcagttcgg tgtaggtcgt tcgctccaag 3360
ctgggctgtg tgcacgaacc ccccgttcag cccgaccgct gcgccttatc cggtaactat 3420
cgtcttgagt ccaacccggt aagacacgac ttatcgccac tggcagcagc cactggtaac 3480
aggattagca gagcgaggta tgtaggcggt gctacagagt tcttgaagtg gtggcctaac 3540
tacggctaca ctagaagaac agtatttggt atctgcgctc tgctgaagcc agttaccttc 3600
ggaaaaagag ttggtagctc ttgatccggc aaacaaacca ccgctggtag cggttttttt 3660
gtttgcaagc agcagattac gcgcagaaaa aaaggatctc aagaagatcc tttgatcttt 3720
tctacggggt ctgacgctca gtggaacgaa aactcacgtt aagggatttt ggtcatgaga 3780
ttatcaaaaa ggatcttcac ctagatcctt ttaaattaaa aatgaagttt taaatcaatc 3840
taaagtatat atgagtaaac ttggtctgac agttaccaat gcttaatcag tgaggcacct 3900
atctcagcga tctgtctatt tcgttcatcc atagttgcct gactccccgt cgtgtagata 3960
actacgatac gggagggctt accatctggc cccagtgctg caatgatacc gcgagaccca 4020
cgctcaccgg ctccagattt atcagcaata aaccagccag ccggaagggc cgagcgcaga 4080
agtggtcctg caactttatc cgcctccatc cagtctatta attgttgccg ggaagctaga 4140
gtaagtagtt cgccagttaa tagtttgcgc aacgttgttg ccattgctac aggcatcgtg 4200
gtgtcacgct cgtcgtttgg tatggcttca ttcagctccg gttcccaacg atcaaggcga 4260
gttacatgat cccccatgtt gtgcaaaaaa gcggttagct ccttcggtcc tccgatcgtt 4320
gtcagaagta agttggccgc agtgttatca ctcatggtta tggcagcact gcataattct 4380
cttactgtca tgccatccgt aagatgcttt tctgtgactg gtgagtactc aaccaagtca 4440
ttctgagaat agtgtatgcg gcgaccgagt tgctcttgcc cggcgtcaat acgggataat 4500
accgcgccac atagcagaac tttaaaagtg ctcatcattg gaaaacgttc ttcggggcga 4560
aaactctcaa ggatcttacc gctgttgaga tccagttcga tgtaacccac tcgtgcaccc 4620
aactgatctt cagcatcttt tactttcacc agcgtttctg ggtgagcaaa aacaggaagg 4680
caaaatgccg caaaaaaggg aataagggcg acacggaaat gttgaatact catactcttc 4740

ctttttcaat attattgaag catttatcag ggttattgtc tcatgagcgg atacatattt    4800
gaatgtattt agaaaaataa acaaataggg gttccgcgca catttccccg aaaagtgcca    4860
cctgacgtc                                                            4869

<210>  34
<211>  20
<212>  DNA
<213>  Artificial

<220>
<223>  An artificially synthesized primer sequence

<400>  34
ggggatcagc gtttgagtaa                                                20

<210>  35
<211>  20
<212>  DNA
<213>  Artificial

<220>
<223>  An artificially synthesized primer sequence

<400>  35
taggccccac ctccttctat                                                20

<210>  36
<211>  30
<212>  DNA
<213>  Artificial

<220>
<223>  An artificially synthesized primer sequence

<400>  36
tgcggatcca gagcagattg tactgagagt                                     30

<210>  37
<211>  29

<212>  DNA
<213>  Artificial

<220>
<223>  An artificially synthesized primer sequence

<400>  37
ctctatctcg agtgaggcgg aaagaacca                                          29


<210>  38
<211>  40
<212>  DNA
<213>  Artificial

<220>
<223>  An artificially synthesized primer sequence

<400>  38
tttaagcttg aagactattt ttacatcagg ttgtttttct                              40


<210>  39
<211>  37
<212>  DNA
<213>  Artificial

<220>
<223>  An artificially synthesized primer sequence

<400>  39
tttaagcttg aagacacggt gtttcgtcct ttccaca                                 37


<210>  40
<211>  51
<212>  DNA
<213>  Artificial

<220>
<223>  An artificially synthesized double-stranded oligonucleotide seque
       nce

<400> 40
caccgaagca gcacgacttc ttcttcaaga gagaagaagt cgtgctgctt c 51

<210> 41
<211> 51
<212> DNA
<213> Artificial

<220>
<223> An artificially synthesized double-stranded oligonucleotide seque
nce

<400> 41
aaaagaagca gcacgacttc ttctctcttg aagaagaagt cgtgctgctt c 51

<210> 42
<211> 30
<212> DNA
<213> Artificial

<220>
<223> An artificially synthesized primer sequence

<400> 42
ggggtaccag gatggagccg ctgaaggtgg 30

<210> 43
<211> 33
<212> DNA
<213> Artificial

<220>
<223> An artificially synthesized primer sequence

<400> 43
gggaattctt aggatgctct gatgttggcg tcg 33

<210> 44
<211> 51

<212>  DNA
<213>  Artificial

<220>
<223>  An artificially synthesized hairpin siRNA sequence

<400>  44
aaacttatgg atggagcacc tttcaagaga aggtgctcca tccataagtt t                51


<210>  45
<211>  51
<212>  DNA
<213>  Artificial

<220>
<223>  An artificially synthesized hairpin siRNA sequence

<400>  45
aatcagagaa gctttactca tttcaagaga atgagtaaag cttctctgat t                51


<210>  46
<211>  51
<212>  DNA
<213>  Artificial

<220>
<223>  An artificially synthesized hairpin siRNA sequence

<400>  46
aacaaactga ctgaagataa gttcaagaga cttatcttca gtcagtttgt t                51


<210>  47
<211>  51
<212>  DNA
<213>  Artificial

<220>
<223>  An artificially synthesized hairpin siRNA sequence

<400>  47

aactcgtaat gacatttcaa cttcaagaga gttgaaatgt cattacgagt t 51

<210> 48
<211> 51
<212> DNA
<213> Artificial

<220>
<223> An artificially synthesized hairpin siRNA sequence

<400> 48
aaaagtgatc tgcaactctt tttcaagaga aaagagttgc agatcacttt t 51

<210> 49
<211> 51
<212> DNA
<213> Artificial

<220>
<223> An artificially synthesized hairpin siRNA sequence

<400> 49
aagtgatctg caactctttc attcaagaga tgaaagagtt gcagatcact t 51

<210> 50
<211> 51
<212> DNA
<213> Artificial

<220>
<223> An artificially synthesized hairpin siRNA sequence

<400> 50
aactctttca ccatctgtaa tttcaagaga attacagatg gtgaaagagt t 51

<210> 51
<211> 51
<212> DNA
<213> Artificial

<220>
<223>  An artificially synthesized hairpin siRNA sequence

<400>  51
aactgttcga ttgtgttcaa tttcaagaga attgaacaca atcgaacagt t          51


<210>  52
<211>  51
<212>  DNA
<213>  Artificial

<220>
<223>  An artificially synthesized hairpin siRNA sequence

<400>  52
aaggatgctg atatgctaac tttcaagaga agttagcata tcagcatcct t          51


<210>  53
<211>  51
<212>  DNA
<213>  Artificial

<220>
<223>  An artificially synthesized hairpin siRNA sequence

<400>  53
aactggtgat gagcaagtat gttcaagaga catacttgct catcaccagt t          51


<210>  54
<211>  51
<212>  DNA
<213>  Artificial

<220>
<223>  An artificially synthesized hairpin siRNA sequence

<400>  54
aagtatggaa ggaagttcaa gttcaagaga cttgaacttc cttccatact t          51

<210> 55
<211> 51
<212> DNA
<213> Artificial

<220>
<223> An artificially synthesized hairpin siRNA sequence

<400> 55
aacatctacc agctgaaggt gttcaagaga caccttcagc tggtagatgt t 51

<210> 56
<211> 51
<212> DNA
<213> Artificial

<220>
<223> An artificially synthesized hairpin siRNA sequence

<400> 56
aagcaatgaa gaatctgaga cttcaagaga gtctcagatt cttcattgct t 51

<210> 57
<211> 21
<212> DNA
<213> Artificial

<220>
<223> An artificially synthesized target sequence for siRNA

<400> 57
aaacttatgg atggagcacc t 21

<210> 58
<211> 21
<212> DNA
<213> Artificial

<220>

<223>  An artificially synthesized target sequence for siRNA

<400>  58
aatcagagaa gctttactca t                                                21


<210>  59
<211>  21
<212>  DNA
<213>  Artificial

<220>
<223>  An artificially synthesized target sequence for siRNA

<400>  59
aacaaactga ctgaagataa g                                                21


<210>  60
<211>  21
<212>  DNA
<213>  Artificial

<220>
<223>  An artificially synthesized target sequence for siRNA

<400>  60
aactcgtaat gacatttcaa c                                                21


<210>  61
<211>  21
<212>  DNA
<213>  Artificial

<220>
<223>  An artificially synthesized target sequence for siRNA

<400>  61
aaaagtgatc tgcaactctt t                                                21


<210>  62

<211>  21
<212>  DNA
<213>  Artificial

<220>
<223>  An artificially synthesized target sequence for siRNA

<400>  62
aagtgatctg caactctttc a                                                21


<210>  63
<211>  21
<212>  DNA
<213>  Artificial

<220>
<223>  An artificially synthesized target sequence for siRNA

<400>  63
aactctttca ccatctgtaa t                                                21


<210>  64
<211>  21
<212>  DNA
<213>  Artificial

<220>
<223>  An artificially synthesized target sequence for siRNA

<400>  64
aactgttcga ttgtgttcaa t                                                21


<210>  65
<211>  21
<212>  DNA
<213>  Artificial

<220>
<223>  An artificially synthesized target sequence for siRNA

<400>  65
aaggatgctg atatgctaac t                                                21

<210>  66
<211>  21
<212>  DNA
<213>  Artificial

<220>
<223>  An artificially synthesized target sequence for siRNA

<400>  66
aactggtgat gagcaagtat g                                                21

<210>  67
<211>  21
<212>  DNA
<213>  Artificial

<220>
<223>  An artificially synthesized target sequence for siRNA

<400>  67
aagtatggaa ggaagttcaa g                                                21

<210>  68
<211>  21
<212>  DNA
<213>  Artificial

<220>
<223>  An artificially synthesized target sequence for siRNA

<400>  68
aacatctacc agctgaaggt g                                                21

<210>  69
<211>  21
<212>  DNA

<213>  Artificial

<220>
<223>  An artificially synthesized target sequence for siRNA

<400>  69
aagcaatgaa gaatctgaga c                                                21